# EUROPEAN PATENT APPLICATION

(11) **EP 3 970 801 A1**
(43) Date of publication of application: **23.03.2022**
(21) Application number: 20805621.8
(22) Date of filing: 12.05.2020
(51) Int. Cl.: A61P 35/00, A61P 43/00, C07K 7/64, A61K 38/08

(54) **RAS INHIBITORY PEPTIDE**

(30) Priority: 14.05.2019 JP 2019091419
(71) Applicant: Ichimaru Pharcos Co., Ltd., Motosu-shi, Gifu 501-0475 (JP)
(72) Inventor: SAKAMOTO Kotaro, Motosu-shi, Gifu 501-0475 (JP)
(74) Representative: Winter, Brandl - Partnerschaft mbB
(86) International application number: PCT/JP2020/018956
(87) International publication number: WO 2020/230780

(57) **Abstract**

[Problem]

To improve protease-mediated degradability and conformational stability of Ras inhibitory peptides for use as pharmaceuticals, diagnostics, and/or research reagents.

[Solution]

A cyclic peptide comprising an amino acid sequence represented by formula (1):

c[X¹-X²-X³-c(X⁴-X⁵-X⁶-X⁷-X⁸-X⁹-X¹⁰-X¹¹)] (1),

or a pharmaceutically acceptable salt thereof. Herein, in the above formula, X¹, X⁴ and X¹¹, each independently denotes an amino acid residue having amino, carboxy, thiol, allyl, alkynyl, azide or halogen groups, or derivatives thereof. X³, X⁵, and X⁷, each independently denotes an amino acid residue with a hydrocarbon group that may have a substituent, an amino acid residue with an aromatic carbocyclic group that may have a substituent, or an amino acid residue with an aromatic heterocyclic group that may have a substituent, or derivatives thereof. X⁸ is an aspartic acid residue or a derivative thereof, and X², X⁶, X⁹, and X¹⁰ are any amino acid residues.

SEQUENCE LISTING

<110>  ICHIMARU PHARCOS Co., Ltd.

<120>  Ras Inhibitory Peptide

<130>  PW20004ICH-PCT

<150>  JP 2019-091419
<151>  2019-05-14

<160>  23

<170>  PatentIn version 3.5

<210>  1
<211>  11
<212>  PRT
<213>  Artificial sequence

<220>
<223>  Seq-1, amide(1-11)/DIE(4-11)

<220>
<221>  MOD_RES
<222>  (1)..(1)
<223>  bAla

<220>
<221>  MISC_FEATURE
<222>  (1)..(11)
<223>  Cyclization by amide bond (amide 1-11)

<220>
<221>  MOD_RES
<222>  (3)..(3)
<223>  Nle

<220>
<221>  MISC_FEATURE
<222>  (4)..(11)
<223>  Thiol groups of side chains are linked by 1,2-diiodoethane (DIE)

<220>
<221>  MOD_RES
<222>  (7)..(7)
<223>  4-fluoro-L-Phenylalanine (4fF)

<400>  1

Xaa Pro Xaa Cys Ile Ser Xaa Asp Pro Val Cys
1               5                   10

<210>  2
<211>  11
<212>  PRT
<213>  Artificial sequence

<220>
<223>  Seq-2, amide(1-11)/DIP(4-11)

<220>
<221> MOD_RES
<222> (1)..(1)
<223> bAla

<220>
<221> MISC_FEATURE
<222> (1)..(11)
<223> Cyclization by amide bond (amide 1-11)

<220>
<221> MOD_RES
<222> (3)..(3)
<223> Nle

<220>
<221> MISC_FEATURE
<222> (4)..(11)
<223> Thiol groups of side chains are linked by 1,3-diiodopropane (DIP)

<220>
<221> MOD_RES
<222> (7)..(7)
<223> 4-fluoro-L-Phenylalanine (4fF)

<400> 2

Xaa Pro Xaa Cys Ile Ser Xaa Asp Pro Val Cys
1               5                   10


<210> 3
<211> 11
<212> PRT
<213> Artificial sequence

<220>
<223> Seq-3, amide(1-11)/DIB(4-11)


<220>
<221> MOD_RES
<222> (1)..(1)
<223> bAla

<220>
<221> MISC_FEATURE
<222> (1)..(11)
<223> Cyclization by amide bond (amide 1-11)

<220>
<221> MOD_RES
<222> (3)..(3)
<223> Nle

<220>
<221> MISC_FEATURE
<222> (4)..(11)
<223> Thiol groups of side chains are linked by 1,3-diiodobutane (DIB)

<220>
<221> MOD_RES

<222>  (7)..(7)
<223>  4-fluoro-L-Phenylalanine (4fF)

<400>  3

Xaa Pro Xaa Cys Ile Ser Xaa Asp Pro Val Cys
1               5                   10


<210>  4
<211>  11
<212>  PRT
<213>  Artificial sequence

<220>
<223>  Seq-4,  amide(1-11)/DIE(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodoethane (DIE)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  4

Xaa Pro Xaa Cys Ile Ser Xaa Asp Pro Val Cys
1               5                   10


<210>  5
<211>  11
<212>  PRT
<213>  Artificial sequence

<220>
<223>  Seq-5, amide(1-11)/DIP(4-11)

<220>
<221> MISC_FEATURE
<222> (1)..(11)
<223> Cyclization by amide bond (amide 1-11)

<220>
<221> MOD_RES
<222> (1)..(1)
<223> bAla

<220>
<221> MOD_RES
<222> (3)..(3)
<223> Nle

<220>
<221> MISC_FEATURE
<222> (4)..(11)
<223> Thiol groups of side chains are linked by 1,3-diiodopropane (DIP)

<220>
<221> MOD_RES
<222> (7)..(7)
<223> 4-fluoro-L-Phenylalanine (4fF)

<220>
<221> MISC_FEATURE
<222> (11)..(11)
<223> D-form optical isomer

<400> 5

Xaa Pro Xaa Cys Ile Ser Xaa Asp Pro Val Cys
1               5                   10


<210> 6
<211> 11
<212> PRT
<213> Artificial sequence

<220>
<223> Seq-6, amide(1-11)/DIB(4-11)


<220>
<221> MISC_FEATURE
<222> (1)..(11)
<223> Cyclization by amide bond (amide 1-11)

<220>
<221> MOD_RES
<222> (1)..(1)
<223> bAla

<220>
<221> MOD_RES
<222> (3)..(3)
<223> Nle

<220>
<221> MISC_FEATURE
<222> (4)..(11)

<223>  Thiol groups of side chains are linked by 1,3-diiodobutane (DIB)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  6

Xaa Pro Xaa Cys Ile Ser Xaa Asp Pro Val Cys
1               5                   10


<210>  7
<211>  11
<212>  PRT
<213>  Artificial sequence

<220>
<223>  Seq-7, amide(1-11)/DIE(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodoethane (DIE)

<220>
<221>  MOD_RES
<222>  (7)..(7)
<223>  4-fluoro-L-Phenylalanine (4fF)

<400>  7

Xaa Pro Xaa Cys Ile Ser Xaa Asp Pro Val Cys
1               5                   10


<210>  8
<211>  11
<212>  PRT
<213>  Artificial sequence

<220>
<223>  Seq-8, amide(1-11)/DIP(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (7)..(7)
<223>  4-fluoro-L-Phenylalanine (4fF)

<400>  8

Xaa Pro Xaa Cys Ile Ser Xaa Asp Pro Val Cys
1               5                   10

<210>  9
<211>  11
<212>  PRT
<213>  Artificial sequence

<220>
<223>  Seq-9, amide(1-11)/DIB(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodobutane (DIB)

<220>
<221>  MOD_RES
<222>  (7)..(7)
<223>  4-fluoro-L-Phenylalanine (4fF)

<400>  9

Xaa Pro Xaa Cys Ile Ser Xaa Asp Pro Val Cys
1               5                   10


<210>  10
<211>  11
<212>  PRT
<213>  Artificial sequence

<220>
<223>  Seq-10, amide(1-11)/DIE(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodoethane (DIE)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  10

Xaa Pro Xaa Cys Ile Ser Xaa Asp Pro Val Cys
1               5                   10


<210>  11
<211>  11
<212>  PRT
<213>  Artificial sequence

<220>

<223>  Seq-11, amide(1-11)/DIP(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  11

Xaa Pro Xaa Cys Ile Ser Xaa Asp Pro Val Cys
1               5                   10

<210>  12
<211>  11
<212>  PRT
<213>  Artificial sequence

<220>
<223>  Seq-12, amide(1-11)/DIB(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  gamma-aminobutyric acid (gamma-Aba)

<220>
<221>  MOD_RES
<222>  (3)..(3)
<223>  Nle

<220>
<221> MISC_FEATURE
<222> (4)..(11)
<223> Thiol groups of side chains are linked by 1,3-diiodobutane (DIB)

<220>
<221> MOD_RES
<222> (7)..(7)
<223> 4-fluoro-L-Phenylalanine (4fF)

<220>
<221> MISC_FEATURE
<222> (11)..(11)
<223> D-form optical isomer

<400> 12

Xaa Pro Xaa Cys Ile Ser Xaa Asp Pro Val Cys
1               5                   10


<210> 13
<211> 11
<212> PRT
<213> Artificial sequence

<220>
<223> Seq-13, amide(1-11)/DIP(4-11)


<220>
<221> MISC_FEATURE
<222> (1)..(11)
<223> Cyclization by amide bond (amide 1-11)

<220>
<221> MOD_RES
<222> (1)..(1)
<223> bAla

<220>
<221> MOD_RES
<222> (3)..(3)
<223> Nle

<220>
<221> MISC_FEATURE
<222> (4)..(11)
<223> Thiol groups of side chains are linked by 1,3-diiodopropane (DIP)

<220>
<221> MOD_RES
<222> (5)..(5)
<223> L-Phenylglycine (Phg)

<220>
<221> MOD_RES
<222> (7)..(7)
<223> 4-fluoro-L-Phenylalanine (4fF)

<220>
<221> MISC_FEATURE
<222> (11)..(11)

<223>  D-form optical isomer

<400>  13

Xaa Pro Xaa Cys Xaa Ser Xaa Asp Pro Val Cys
1               5                   10


<210>  14
<211>  11
<212>  PRT
<213>  Artificial sequence

<220>
<223>  Seq-14, amide(1-11)/DIP(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (5)..(5)
<223>  2-Aminooctanoic acid (Aoc)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  14

Xaa Pro Xaa Cys Xaa Ser Xaa Asp Pro Val Cys
1               5                   10


<210>  15
<211>  11
<212>  PRT
<213>  Artificial sequence

<220>
<223>  Seq-15, amide(1-11)/DIP(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (5)..(5)
<223>  2-Aminononanonic acid (Anon)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  15

Xaa Pro Xaa Cys Xaa Ser Xaa Asp Pro Val Cys
1               5                   10

<210>  16
<211>  11
<212>  PRT
<213>  Artificial sequence

<220>
<223>  Seq-16, amide(1-11)/DIP(4-11)

<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (5)..(5)
<223>  2-Aminononanonic acid (Anon)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  16

Xaa Pro Xaa Cys Xaa Ser Xaa Asp Pro Phe Cys
1               5                   10


<210>  17
<211>  11
<212>  PRT
<213>  Artificial sequence

<220>
<223>  Seq-17, amide(1-11)/DIP(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES

<222>  (5)..(5)
<223>  2-Aminononanonic acid (Anon)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  17

Xaa Pro Xaa Cys Xaa Ser Xaa Asp Pro Trp Cys
1               5                   10


<210>  18
<211>  11
<212>  PRT
<213>  Artificial sequence

<220>
<223>  Seq-18, amide(1-11)/DIP(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (5)..(5)
<223>  2-Aminononanonic acid (Anon)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  18

Xaa Pro Xaa Cys Xaa Ser Xaa Asp Pro Xaa Cys
1               5                   10


<210>  19
<211>  11
<212>  PRT
<213>  Artificial sequence

<220>
<223>  Seq-19, amide(1-11)/DIP(4-11)


<220>
<221>  MISC_FEATURE
<222>  (1)..(11)
<223>  Cyclization by amide bond (amide 1-11)

<220>
<221>  MOD_RES
<222>  (1)..(1)
<223>  bAla

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodopropane (DIP)

<220>
<221>  MOD_RES
<222>  (5)..(5)
<223>  2-Aminononanonic acid (Anon)

<220>
<221>  MOD_RES
<222>  (7)..(7)
<223>  4-fluoro-L-Phenylalanine (4fF)

<220>
<221>  MISC_FEATURE
<222>  (11)..(11)
<223>  D-form optical isomer

<400>  19

Xaa Pro Xaa Cys Xaa Ser Xaa Asp Pro Trp Cys
1               5                   10


<210>  20
<211>  11
<212>  PRT
<213>  Artificial Sequence

<220>
<223>  LO2

<220>
<221> MOD_RES
<222> (1)..(1)
<223> bAla

<220>
<221> MISC_FEATURE
<222> (1)..(11)
<223> Cyclization by amide bond (amide 1-11)

<220>
<221> MOD_RES
<222> (3)..(3)
<223> Nle

<220>
<221> MISC_FEATURE
<222> (4)..(11)
<223> Thiol groups of side chains are linked by 1,2-diiodoethane (DIE)

<220>
<221> MOD_RES
<222> (5)..(5)
<223> 2-amino octanoic acid (Aoc)

<220>
<221> MOD_RES
<222> (7)..(7)
<223> 4-fluoro-Phenylalanine (4fF)

<220>
<221> MOD_RES
<222> (10)..(10)
<223> MeVal

<220>
<221> MISC_FEATURE
<222> (11)..(11)
<223> D-form optical isomer

<400> 20

Xaa Pro Xaa Cys Xaa Ser Phe Asp Pro Val Cys
1               5                   10


<210> 21
<211> 11
<212> PRT
<213> Artificial Sequence

<220>
<223> LO3


<220>
<221> MOD_RES
<222> (1)..(1)
<223> bAla

<220>

<221>  MISC_FEATURE
<222>  (1)..(11)
<223>  Cyclization by amide bond (amide 1-11)

<220>
<221>  MOD_RES
<222>  (3)..(3)
<223>  Nle

<220>
<221>  MISC_FEATURE
<222>  (4)..(11)
<223>  Thiol groups of side chains are linked by 1,3-diiodobutane (DIB)

<220>
<221>  MOD_RES
<222>  (5)..(5)
<223>  2-amino octanoic acid (Aoc)

<220>
<221>  MOD_RES
<222>  (7)..(7)
<223>  4-fluoro-Phenylalanine (4fF)

<220>
<221>  MOD_RES
<222>  (10)..(10)
<223>  MeVal

<220>
<221>  MISC_FEATURE
<222>  (11)..(11)
<223>  D-form optical isomer

<400>  21

Xaa Pro Xaa Cys Xaa Ser Phe Asp Pro Val Cys
1               5                   10


<210>  22
<211>  19
<212>  PRT
<213>  Artificial Sequence

<220>
<223>  K-Ras(G12D)-selective inhibitory peptide


<220>
<221>  MOD_RES
<222>  (1)..(1)
<223>  ACETYLATION

<220>
<221>  DISULFID
<222>  (5)..(15)

<220>
<221>  MOD_RES
<222>  (19)..(19)
<223>  AMIDATION

<400>  22

Arg Arg Arg Arg Cys Pro Leu Tyr Ile Ser Tyr Asp Pro Val Cys Arg
1               5                   10                  15

Arg Arg Arg

<210>  23
<211>  11
<212>  PRT
<213>  Artificial Sequence

<220>
<223>  Biotin-BC1

<220>
<221>  MOD_RES
<222>  (1)..(1)
<223>  bAla

<220>
<221>  MISC_FEATURE
<222>  (1)..(11)
<223>  Cyclization by amide bond (amide 1-11)

<220>
<221>  MOD_RES
<222>  (3)..(3)
<223>  Nle

<220>
<221>  DISULFID
<222>  (4)..(11)

<220>
<221>  MISC_FEATURE
<222>  (4)..(4)
<223>  D-form optical isomer

<220>
<221>  MOD_RES
<222>  (5)..(5)
<223>  2-amino octanoic acid (Aoc)

<220>
<221>  MOD_RES
<222>  (7)..(7)
<223>  N-methyl-4-fluoro-Phenylalanine (4fF)

<220>
<221>  MOD_RES
<222>  (9)..(9)
<223>  epsilon-NH-Biotin

<220>
<221>  MOD_RES
<222>  (10)..(10)
<223>  MeVal

<220>
<221>  MISC_FEATURE
<222>  (11)..(11)
<223>  D-form optical isomer

<400>  23

Xaa Pro Xaa Cys Xaa Ser Phe Asp Lys Val Cys
1               5                   10

## Description

### Cross reference

This application claims priority based on Japanese Patent Application No. 2019-091419 filed in Japan on May 14, 2019, the entire contents of which are incorporated herein by reference in their entirety. In addition, all the contents described in all patents, patent applications, and documents cited in the present application are incorporated herein by reference in their entirety.

### Technical field

The present invention relates to a peptide and the like that inhibits the activity of Ras, and more particularly, relates to a cyclic peptide and the like having a specific amino acid sequence and a cyclic structure.

### Background art

The Ras protein (meaning the Ras subfamily, hereinafter abbreviated as Ras) is an intracellularly expressed GTPase that functions as a molecular switch to turn off (inactivate) and turn on (activate) cell growth signaling by binding to GDP or GTP. The GDP-bound form is in the off state, while the GTP-bound form is in the on state. A guanine nucleotide exchange factor (GEF) induced by signals from cell surface receptors converts Ras from the GDP-bound form to the GTP-bound form, and the GTP-bound Ras further transmits signals by interacting with downstream proteins such as RAF and PI3K. The GTP bound to Ras is hydrolyzed from GTP to GDP by Ras's own enzymatic activity with the help of GTPase activating protein (GAP) .

Ras consists of 188-189 amino acid sequences, and any amino acid mutation greatly suppresses its own GTPase activity and the hydrolysis of GTP by GAP, resulting in a GTP-binding bias. This results in the prolongation of the cell proliferation signal.

Approximately 30% of human tumors express mutant Ras with amino acid mutations, which is used as a driver for tumor growth (Non-Patent Literature 1). Among Ras proteins, K-Ras, N-Ras, and H-Ras are examples of members who are attracting attention as drug discovery targets. Among them, amino acid mutations of K-Ras are known to occur at the highest frequency of about 20% of human tumors (Non-Patent Literature 1).

It has been reported that the majority of the positions of amino acid mutation in K-Ras are glycine at position 12, followed by glycine at position 13, glutamine at position 61, and other positions. The most frequent mutations at position 12 glycine are aspartic acid (about 30%), followed by valine (about 20%), cysteine (about 10%), alanine (about 5%), serine (about 5%), arginine (about 3%), and other amino acids (Non-Patent Literature 2).

These indicate that K-Ras(G12D), a K-Ras in which glycine at position 12 is mutated to aspartic acid, is the most important drug target in cancer therapy. In 2017, the inventor has found and reported that a cyclic peptide, KRpep-2d:
Ac-Arg-Arg-Arg-Arg-c(Cys-Pro-Leu-Tyr-Ile-Ser-Tyr-Asp-Pro-Val-Cys )-Arg-Arg-Arg-Arg-NH₂ (SEQ ID No.22) selectively binds to K-Ras(G12D) over the wild-type K-Ras in a cell-free assay, and inhibits the conversion of GDP-bound K-Ras(G12D) to GTP-bound K-Ras(G12D) (Non-Patent Literature 3). KRpep-2d was shown to inhibit significantly and selectively the growth of A427 cells expressing K-Ras(G12D) in cell testing. The inventor also analyzed the crystal structure of the complex of KRpep-2d and K-Ras (G12D) and reported the mechanism of binding (Non-Patent Literature 4).

### Citation list

### Non-patent literature

[Non-Patent Literature 1] Takashima A and Faller DV. Expert Opin Ther Targets. 2013, 17(5):507-531.
[Non-Patent Literature 2] Pender A, Garcia-Murillas I, Rana S, Cutts RJ, Kelly G, Fenwick K, Kozarewa I, Gonzalez de Castro D, Bhosle J, O'Brien M, Turner NC, Popat S, and Downward J. PLoS One. 2015, 10(9):e0139074.
[Non-Patent Literature 3] Sakamoto K, Kamada Y, Sameshima T, Yaguchi M, Niid, Sasaki S, Miwa M, Ohkubo S, Sakamoto JI, Kamaura M, Cho N, and Tani A. Biochem Biophys Res Commun. 2017, 484(3):605-611.
[Non-Patent Literature 4] Sogabe S, Kamada Y, Miwa M, Niid, Sameshima T, Kamaura M, Yonemori K, Sasaki S, Sakamoto JI, and Sakamoto K. ACS Med Chem Lett. 2017, 8(7):732-736.
[Non-Patent Literature 5] Niida A, Sasaki S, Yonemori K, Sameshima T, Yaguchi M, Asami T, Sakamoto K, and Kamaura M. Bioorg Med Chem Lett. 2017, 27(12):2757-2761.
[Non-Patent Literature 6] Fairlie DP and Dantas de Araujo. Biopolymers 2016, 106(6):843-852.
[Non-Patent Literature 7] Lau YH, de Andrade P, Wu Y, and Spring DR. Chem Soc Rev. 2015, 44(1)91-102.

### Summary of invention

### Technical problem

According to the analysis of the present inventor, there are some problems in using KRpep-2d as a pharmaceutical product, a diagnostic agent, and/or a research reagent as shown below. (1) There is a concern that KRpep-2d, which consists only of natural amino acids, has low resistance to protease degradation. (2) Since KRpep-2d is cyclized by a disulfide bond between the side chains of the two cysteines (Cys) in the sequence, the disulfide bonds may be cleaved under reducing conditions in the cell, and the cyclic structure of the peptide may not be maintained, resulting in reduced binding and inhibitory activity against Ras. In fact, the inventor has found that the inhibitory activity of KRpep-2d against the exchange reaction from GDP-bound to GTP-bound form of K-Ras(G12D) is attenuated more than 50-fold in cell-free tests under reducing conditions including dithiothreitol (DTT), compared to the inhibitory activity under non-reducing conditions (Non-Patent Literatures 3 and 5). (3) The significant growth inhibitory activity of KRpep-2d in cell tests is in the range of several to several tens of µM (Non-Patent Literatures 3 and 5), and there is room for improvement in its use as a drug, diagnostic agent, and/or research reagent.

The present invention has been made in view of such problems, and its object is to provide a novel Ras inhibitory peptide that can improve at least one of the above problems for use of Ras inhibitory peptides as pharmaceuticals, diagnostics, and/or research reagents.

### Solution to problem

The present inventor discovered a novel group of Ras inhibitory peptides while conducting optimization studies of KRpep-2d. In order to solve the problems of KRpep-2d, based on the structural information of the complex of KRpep-2d and K-Ras(G12D) described in the non-patent Literature 4, the inventor predicted amino acid substitutions that would improve the functions of KRpep-2d such as Ras-binding activity, resistance to protease degradation and cell growth inhibitory activity. KRpep-2d is characterized by (1) being circularized by a disulfide bond between the side chains of Cys at position 5 and Cys at position 15, (2) amino acids at positions from 6 to 14 mainly interact with K-Ras(G12D) as a pharmacophore, (3) the amino acids at positions from 1 to 4 and those from 16 to 19 function as a cell-penetrating peptide (CPP), but also contribute to the conformational regulation of the peptide, since deletion of these amino acids greatly reduces the binding and inhibitory activity of the peptide against Ras (Non-Patent Literatures 4 and 5). Thus, the inventor tried to design the following molecular structures: (1) in order to maintain Ras-binding activity even under reducing conditions in the cell, the disulfide bond between the side chains of Cys at position 5 and Cys at position 15 is replaced with a bond that does not readily cleave under reducing conditions, (2) introduction of unnatural amino acid structures at positions from 6 to 14 to enhance the interaction with Ras, and (3) to reduce the molecular weight, deletion of amino acids at positions from 1 to 4 and those from 16 to 19 to lower the molecular weight still results in Ras-binding activity. During these optimization studies of KRpep-2d, a group of novel Ras inhibitory peptide sequences with Ras-binding activity, resistance to protease degradation, and cell growth inhibitory activity, have been found, which leads to the completion of the present invention.

That is, one aspect of the present invention is a cyclic peptide comprising an amino acid sequence represented by the following formula (1):

c[X¹-X²-X³-c(X⁴-X⁵-X⁶-X⁷-X⁸-X⁹-X¹⁰-X¹¹)] (1)

, or a pharmaceutically acceptable salt thereof. In the above formula, X¹, X⁴ and X¹¹, each independently denotes an amino acid residue having an amino group, a carboxyl group, a thiol group, an allyl group, an alkynyl group, an azido group or a halogen atom, or a derivative thereof. X³, X⁵ and X⁷, each independently denotes an amino acid residue having a hydrocarbon group optionally having a substituent, or an aromatic heterocyclic group optionally having a substituent, or a derivative thereof. X⁸ denotes an aspartic acid residue or a derivative thereof, and X², X⁶, X⁹ and X¹⁰, each denotes any amino acid residue.

Then, X¹ and X¹¹, and X⁴ and X¹¹, independently of each other, covalently bind directly or via a linker to form two cyclic structures in the peptide molecule of formula (1). The covalent bond between X¹ and X¹¹ is any linkage between main-chains, a main-chain and a side-chain, a side-chain and a main-chain or side-chains thereof; and the covalent bond between X⁴ and X¹¹ is any linkage between a side-chain and a main-chain or side-chains thereof.

The preferred forms of each amino acid residue of X¹ to X¹¹ in the peptide of formula (1) above are described in detail in the following embodiments, each of which may be independently and arbitrarily combined with each other.

### Advantageous effects of invention

The present invention provides novel cyclic peptides useful for the use of Ras inhibitory peptides as pharmaceuticals, diagnostics, and/or research reagents.

### Brief description of drawings

FIG.1 shows an amino acid sequence involved in functions of KRpep-2d, such as Ras binding activity (pharmacophore), cyclic structure constraint (S-S bond), and intracellular penetration (cell-penetrating peptide: CPP). The structure of the inventive peptide, which is a modification of KRep-2d, is also outlined.
FIG.2 shows an example of amino acids used in the amino acid residue substitution study at each amino acid position in KRpep-2d.
FIG.3 shows an overview of the competitive binding test by ELISA used to compare the binding activity of a peptide with substituted amino acid residues to K-Ras (G12D) with that of KRpep-2d. In the figure, SA indicates streptavidin, B indicates biotin, and HRP indicates horseradish peroxidase.
FIG.4 shows the peptide concentration-dependent binding activity to K-Ras(G12D), of N-terminal biotin-modified KRpep-2d (Biotin-KRpep-2d) .
FIG.5 shows the peptide concentration-dependent binding activities of biotin modified examples of representative peptide of the invention, to wild-type K-Ras, K-Ras (G12D), K-Ras (G12V), K-Ras (G12C), K-Ras (G13D), wild type N-Ras, N-Ras (Q61K), wild type H-Ras, H-Ras (G12V) and H-Ras (Q61L).
FIG.6 shows the results of comparing the resistance of KRpep-2d and those of representative examples of the inventive peptides to protease degradation when mixed with rat plasma.
FIG.7A shows the results of evaluating the growth inhibitory activity of KRpep-2d and those of representative examples of the inventive peptides against A427 cells expressing K-Ras(G12D) by quantification of ATP.
FIG.7B shows the results of evaluating the growth inhibitory activity of the peptide of Seq-17 against PANC-1 cells expressing K-Ras(G12D) by quantification of ATP.
FIG.8 shows the results of tail vein administration of a representative example of the inventive peptides to subcutaneous cancer-bearing mice and evaluation of its anticancer activity.
FIG.9 shows the results of tail vein administration of a representative example of the inventive peptides to orthotopic transplantation model mice and evaluation of its anticancer activity.
FIG.10 shows structural formulas of representative examples of the inventive peptides used in the examples.

### Description of embodiments

Next, a suitable embodiment of the present invention will be described with reference to the drawings. The embodiments described below do not limit the invention according to the claims, and not all of the various elements and combinations thereof described in the embodiments may be essential to the solution of the invention. In addition, the disclosures of all patent and non-patent literatures cited herein are incorporated herein by reference in their entirety.

### (Definition)

A peptide herein refers to two or more amino acids (for example, 2 to 20 amino acids) connected by an amide bond (peptide bond). In accordance with the conventional peptide notation, the left end is the N-terminal (amino terminal) and the right end is the C-terminal (carboxy terminal). The first carbon atom adjacent to the carbonyl group that forms the peptide bond is referred to as Cα carbon.

In the present specification, "any amino acid, or a derivative thereof" is used in its broadest sense and includes, in addition to natural amino acids, artificial amino acids with non-natural structures, chemically synthesized compounds with properties known in the industry to be characteristic of amino acids, as well as carboxylic acids with functional groups. Examples of unnatural amino acids include, but are not limited to, D-amino acids, α/α-disubstituted amino acids whose main chain structure differs from the natural type (e.g. α-methylated amino acids such as 2-aminoisobutyric acid), N-alkyl-amino acids (such as N-methylated amino acids), N-substituted glycine (peptoid), amino acids with elongated main chains (β-homo amino acids and γ-homo amino acids), amino acids whose side chain structure differs from the natural type (such as cyclohexylalanine, allylglycine, 2-(2-pyridyl)-glycine, 3-(1H-benzoimidazol-2-yl)-alanine), amino acids with partially substituted side chains (such as norleucine, diaminopropanoic acid and 3-(2-pyridyl)-alanine), amino acids with an extra functional group on the side chain, amino acids with extra C, alkyl, or methyl groups on the side chain (such as homonorleucine and γ-methyl leucine), amino acids with halogen atoms (F, Cl, Br, I) in the side chain (such as 3-chloroalanine), carboxylic acids with halogen atoms (F, Cl, Br, I) in the side chain (such as 3-chloropropanoic acid), carboxylic acids with functional groups on the side chain (such as 3-butenoic acid), amino acids with an extra N or amino group on the side chain (such as β-azidoalanine and ornithine), amino acids with an extra O or methoxy group on the side chain (such as O-methyl-serine and O-methyl-threonine), amino acids with extra hydroxy groups in the side chain (such as 3-hydroxy-phenylalanine), amino acids with an extra carboxy group (-COOH) in the side chain (such as 3-carboxy-phenylalanine), amino acids with an extra S in the side chain (such as ethionine), amino acids in which the carboxylic acid functional group in the side chain is protected by an ester (such as aspartic acid-4-methyl ester), amino acids in which a thiol group (-S-) in the side chain is oxidized to a sulfinyl group (-S(=O)-) or a sulfonyl group (-S(=O)₂-) (such as methionine sulfoxide), and the like.

In the present specification, a hydrocarbon group which may have a substituent refers to, for example, C₁₋₁₀ alkyl groups such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl; C₂₋₁₀ alkenyl groups, such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl; C₂₋₁₀ alkynyl groups, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 4-methyl-2-pentynyl; C₃₋₁₀ cycloalkyl groups, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, adamantly; C₃₋₁₀ cycloalkenyl groups such as cyclopropenyl, cyclobutenyl, cyclopentenyl, cyclohexenyl, cycloheptenyl, cyclooctenyl, and the like. An aromatic carbon ring group that may have a substituent refers to, for example, a phenyl group or a naphthyl group. An aromatic heterocyclic group that may have a substituent group means but are not limited to, for example, pyridyl, thienyl, furfuryl, pyrrolyl, imidazolyl, pyrazolyl, thiazolyl, isothiazolyl, oxazolyl, isoxazolyl, pyrazinyl, pyrimidinyl, pyridazinyl, 1,2,4-oxadiazolyl 1,2,4-oxadiazolyl, 1,3,4-oxadiazolyl, 1,2,4-thiadiazolyl, 1,3,4-thiadiazolyl, triazolyl, tetrazolyl, triazinyl, and other 5 or 6 membered monocyclic aromatic heterocyclic groups; and also a 8 or 14 membered fused polycyclic (preferably 2 or 3 ring) aromatic heterocyclic group, such as benzofuranyl, benzothienyl, benzimidazolyl, benzoxazolyl, benzoisoxazolyl, benzothiazolyl, benzoisothiazolyl, benzotriazolyl, imidazopyridinyl, thienopyridinyl, flopyridinyl, pyrrolopyridinyl, pyrazolopyridinyl, oxazolopyridinyl oxazolopyridinyl, thiazolopyridinyl, imidazopyrazinyl, imidazopyrimidinyl, thienopyrimidinyl, flopyrimidinyl, pyrrolopyrimidinyl, pyrazolopyrimidinyl, oxazolopyrimidinyl, thiazolopyrimidinyl, pyrazolotriazinyl, naphtho[2,3 -b]thienyl, phenoxathiinyl, indolyl, isoindolyl, 1H-indazolyl, prinyl, isoquinolyl, quinolyl, phthalazinyl, naphthyridinyl, quinoxalinyl, quinazolinyl, cinolinyl, carbazolyl, β -carbolinyl, phenanthridinyl, acridinyl, phenazinyl, phenothiazinyl, phenoxazinyl, and the like.

As used herein, "Ras" means all wild-type and amino acid mutant proteins of the Ras subfamily, including K-Ras, N-Ras, and H-Ras of mammals such as mice, rats, dogs, monkeys, and humans. The term "Ras" encompasses both GDP-binding and GTP-binding forms.

As used herein, the phrase, "having Ras inhibitory activity" means that, for example, in *in vitro* studies, the peptides of the invention (1) inhibit the binding of the previously reported biotin-labeled form of the Ras inhibitory peptide KRpep-2d to Ras proteins, (2) bind to Ras proteins in a concentration-dependent manner, (3) inhibit the phosphorylation of Erk in Ras-expressing cells, or (4) inhibit the proliferation of Ras-expressing cells. When any one of these effects is shown, the phrase, "having Ras inhibitory activity" is used. The presence or absence of the Ras inhibitory activity can be confirmed according to methods known to those skilled in the art. Methods for confirming the inhibitory activity include, but are not limited to, a method for evaluating the reaction in which GDP on the Ras protein is exchanged for GTP (Non-Patent Literature 3), a method for co-culturing Ras-expressing cells with peptides to determine whether or not Erk, a signal downstream of Ras, is phosphorylated (Non-Patent Literature 3), a method for co-culturing Ras-expressing cells with/without peptides and examining the presence or absence and rate of proliferation of the cells (Non-Patent Literature 3), a method of administering peptides to disease model animals, such as tumor-bearing mice, to determine if and how fast cancer cells are growing.

### (Cyclic peptide)

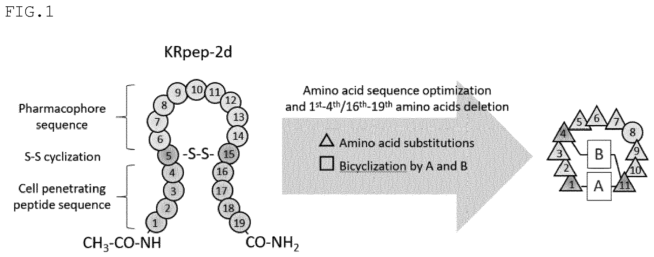

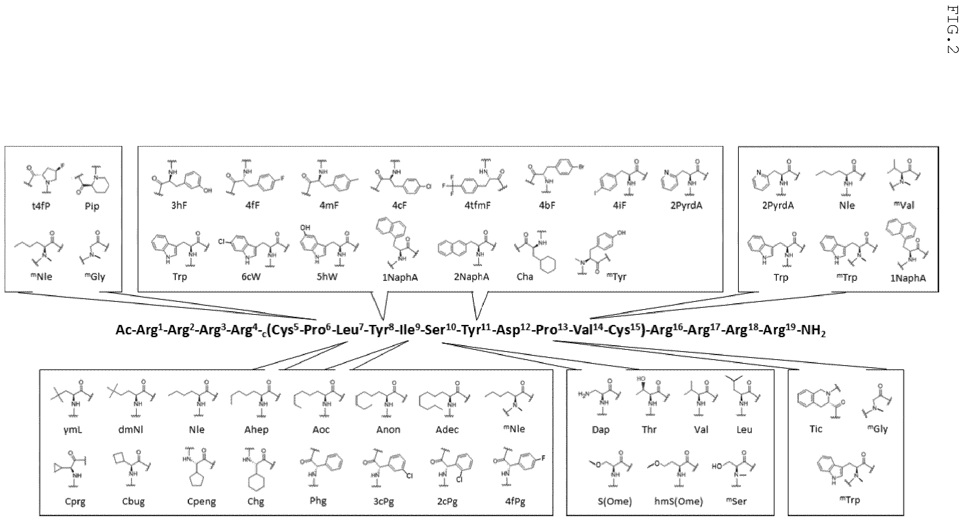

The structure of the cyclic peptide according to one embodiment of the present invention is described with reference to Fig.1. Fig.1 schematically illustrates the relationship between the structures involved in the functions of Ras binding activity (pharmacophore), S-S binding, and CPP of KRpep-2d, which was used as the basis for the design of the cyclic peptide, and the cyclic peptide of this modified form. The amino acid residues from position 6 to position 14, forming the Ras binding site in KRpep-2d, correspond to the amino acid residues from position 2 to position 10 in the cyclic peptide of this embodiment. In addition, the cyclization between the cysteine residues at positions 5 and 15 in KRpep-2d is more stabilized as two cyclic structures: bond A between the amino acid residues at positions 1 and 11, and bond B between the amino acid residues at positions 4 and 11 in this cyclic peptide. Further, examples of amino acids that were used to replace the amino acid residue in each amino acid position of KRpep-2d are shown in Fig. 2.

Therefore, the cyclic peptide in one preferred embodiment of the present invention can be represented by the following formula (1) :

c [X¹-X²-X³-C(X⁴-X⁵-X⁶-X⁷-X⁸-X⁹-X¹⁰-X¹¹)] (1)

Here, each amino acid residue of X¹ to X¹¹ is as follows.
[1] X¹, X⁴ and X¹¹, each independently denotes an amino acid residue having an amino group, a carboxyl group, a thiol group, an allyl group, an alkynyl group, an azido group or a halogen atom, or a derivative thereof. X³, X⁵ and X⁷, each independently denotes an amino acid residue having a hydrocarbon group optionally having a substituent, or an aromatic heterocyclic group optionally having a substituent, or a derivative thereof. X⁸ denotes an aspartic acid residue or a derivative thereof, and X², X⁶, X⁹ and X¹⁰, each denotes any amino acid residue. Each of the amino group and the carboxy group in the "an amino acid residue having an amino group or a carboxy group" may be a group forming an amide bond of the main chain.
   Then, X¹ and X¹¹, and X⁴ and X¹¹, independently of each other, covalently bind directly or via a linker to form two cyclic structures in the peptide molecule of formula (1). The covalent bond between X¹ and X¹¹ is any linkage between main-chains, a main-chain and a side-chain, a side-chain and a main-chain or side-chains thereof. Also, the covalent bond between X⁴ and X¹¹ is any linkage between a side-chain and a main-chain or side-chains thereof.
[2] The linkage between Cα carbon atoms of X¹ and X¹¹ comprises a structure represented by the following formula (2): 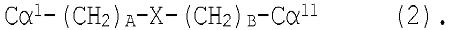 In the above formula, A and B each independently denotes an integer from 0 to 2, a sum of A and B is an integer from 1 or 4, and X is S-S, CH₂-CH₂, S-CH₂, CH₂-S, O-CH₂, CH₂-O, CH=CH, NH-C(=O), N(CH₃)-C (=O), NH-C(=S), N(CH₃)-C(=S), O-C(=O), C(=O)-NH, C(=O)-N(CH₃), C(=S)-NH, C(=S)-N(CH₃), C(=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C(=CH₂)-S, S-(CH₂)₂-S, S-(CH₂)₃-S, S- (CH₂)₄-S, S-CH₂-CH=CH-CH₂-S, S-CH₂-C(=O) -NH, NH-C(=O)-CH₂-S, S-CH₂-C₆H₄-CH₂-S, S-CH₂-C(=O) -CH₂-S or S-CH₂-C(=CH₂)-CH₂-S .
   Among these, it is preferable that X represents NH-C(=O), CH₂-S-CH₂, CH=CH, CH₂-CH₂, and even more preferable when X represents NH-C (=O). The case where X represents NH-C(=O)is, for example, the amide bond formed between the N-terminal amino group of X¹ and the C-terminal carboxy group of X¹¹. The case where X represents CH₂-S-CH₂ includes, for example, the thioether bond formed between the main chain chloro group of 3-chloropropanoic acid at position 1 and the side chain thiol group of cysteine at position 11, or the thioether bond formed by the thiol-ene reaction between the main chain allyl group of 3-butenoic acid at position 1 and the side chain thiol group of cysteine at position 11. The case where X represents CH=CH refers, for example, to the CH=CH bond formed by the olefin metathesis reaction between the allyl group of the main chain of 3-butenoic acid at position 1 and the allyl group of the side chain of β-homo-L-allylglycine at position 11. When this CH=CH bond is reduced, X represents CH₂-CH₂.
[3] The linkage between Cα carbon atoms of X⁴ and X¹¹ comprises a structure represented by the following formula (3): 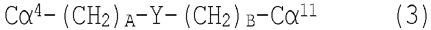 In the formula, A and B each independently denotes an integer from 0 to 4, a sum of A and B is an integer from 2 to 4, and Y is S-S, CH₂-CH₂, S-CH₂, CH₂-S, O-CH₂, CH₂-O, CH=CH, NH-C (=O), N(CH₃)-C(=O), NH-C(=S), N(CH₃)-C(=S), O-C(=O), C(=O)-NH,C(=O) -N(CH₃), C(=S)-NH, C(=S)-N(CH₃), C(=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C(=CH₂)-S, S-(CH₂)₂-S, S-(CH₂)₃-S, S-(CH₂)₄-S, S-CH₂-CH=CH-CH₂-S, S-CH₂-C(=O)-NH, NH-C(=O)-CH₂-S, S-CH₂-C₆H₄-CH₂-S, S-CH₂-C(=O) -CH₂-S S-CH₂-C(=CH₂)-CH₂-S, triazole or dithio-tetrafluorobenzene.
   Among these, it is preferable that Y represents S-S, S-CH₂-S, S-C (=CH₂)-S, S- (CH₂)₂-S, S- (CH₂)₃-S, S-CH₂-C(=CH₂)-CH₂-S, S- (CH₂)₄-S, S-CH₂-CH=CH-CH₂-S, or NH-C(=O), and even more preferably that Y represents S-CH₂-S, S- (CH₂)₂-S, S-(CH₂)₃-S, or S-(CH₂)₄-S. The case where Y represents S-CH₂-S is, for example, when X⁴ and X¹¹ are both cysteines, the -S-CH₂-S- bond formed between the side chain thiol group at position 4 and the diiodomethane (DIM) linker and the side chain thiol group at position 11. The case where Y represents S-(CH₂)₃-S means, for example, the -S-(CH₂)₃-S bond formed between the side chain thiol group of cysteine at position 4 and the 1,3-diiodopropane (DIP) linker and the side chain thiol group of cysteine at position 11. The case where Y represents a triazole is, for example, a triazole-mediated bond formed by a click reaction between the side chains of butenyl-L-glycine at position 4 and β-azido-L-alanine at position 11. It can also be a thioether bond by thiol-ene reaction between allylglycine at position 4 and homocysteine at position 11, or a CH=CH bond by olefin metathesis reaction between allylglycine at position 4 and β-homoallylglycine at position 11. When this CH=CH bond is reduced, Y represents CH₂-CH₂.
[4] X³ and X⁵ are amino acid residues independently represent by any of the following formulas (4) to (7). The amino acid residue of formula (4) is represented by: 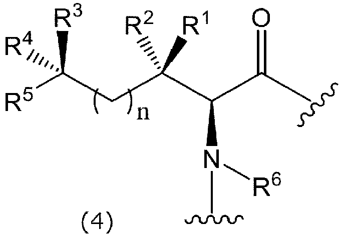 wherein, the wavy line denotes the point of attachment to the carbonyl group or the nitrogen atom that forms the main chain amide bond, and R¹, R² and R⁶ each independently denotes a hydrogen atom or a methyl group. R³, R⁴, and R⁵ each independently denotes a hydrogen atom, a methyl group, or a halogen atom (F, Cl, Br, I), and n denotes an integer of 0 to 10. Among these, leucine, norleucine, isoleucine, valine, 2-aminoheptanoic acid, 2-aminooctanoic acid, 2-aminononanoic acid, 2-aminodecanoic acid, γ-methyl-leucine and 5,5'-dimethylnorleucine, etc. preferable. Among these, leucine, norleucine, isoleucine, valine, 2-aminoheptanoic acid, 2-aminooctanoic acid, 2-aminononanoic acid, 2-aminodecanoic acid, γ-methyl-leucine and 5,5'-dimethylnorleucine, etc. are preferable.
   The amino acid residue of formula (5) is represented by: 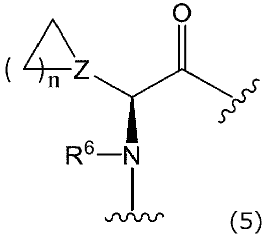 In the formula, Z denotes C or N, R⁶ denotes a hydrogen atom or a methyl group, and n denotes an integer of 1 to 6. Among these, cyclobutylglycine, cyclopentylglycine, cyclohexylglycine and the like are preferable.
   The amino acid residue of formula (6) is represented by: 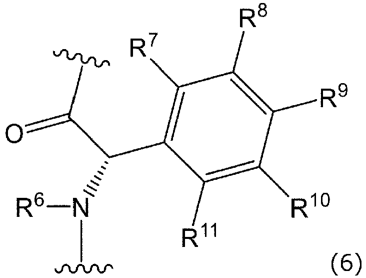 wherein, R⁷, R⁸, R⁹, R¹⁰ and R¹¹ each independently denotes a hydrogen atom or a halogen atom (F, Cl, Br, I), and R⁶ denotes a hydrogen atom or a methyl group. Among these, phenylglycine, 3-chlorophenylglycine, 2-chlorophenylglycine, 4-chlorophenylglycine, 3-fluorophenylglycine, 2-fluorophenylglycine, 4-fluorophenylglycine and the like are preferable.
   The amino acid residue of formula (7) is represented by: 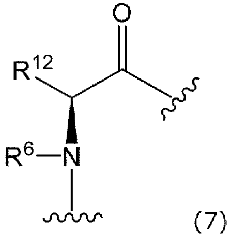 wherein, R¹² denotes a 2-thienyl group, 3-thienyl group, 2-pyridyl group, 3-pyridyl group, 4-pyridyl group, 2-thiazolyl group, 4-thiazolyl group, 2-oxazolyl group, 4-oxazolyl group, 1-pyrazolyl group, 1-imidazolyl group, 1,2,3-triazole-1-yl group, or 1,2,4-triazole-1-yl group. R⁶ denotes a hydrogen atom or a methyl group. The amino acid residue represented by any of these formulas (4) to (7) may be a derivative thereof.
[5] X⁷ is an amino residue represented by any of the following formulas (8) to (11). The amino acid residue of the formula (8) is represented by: 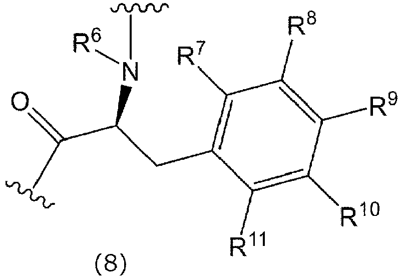 wherein, the wavy line denotes the point of attachment to the carbonyl group or the nitrogen atom that forms the main chain amide bond, and R⁷, R⁸, R⁹, R¹⁰ and R¹¹ each independently denotes a hydrogen atom, a hydroxy group, a methoxy group, a methyl group, a tert-butyl group, a methyl halide group, or a halogen atom (F, Cl, Br, I), and R⁶ denotes a hydrogen atom or a methyl group. Among these, 4-fluorophenylalanine, 4-methylphenylalanine, 4-chlorophenylalanine, 4-trifluoromethyl-phenylalanine, 4-bromophenylalanine, 4-iodophenylalanine and the like are preferable.
   The amino acid residue of formula (9) is represented by: 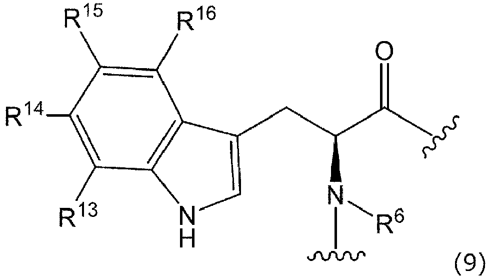 wherein, R¹³, R¹⁴, R¹⁵, R¹⁶ each independently denotes a hydrogen atom, a hydroxy group, a methoxy group, a methyl group, a tert-butyl group, a methyl halide group, or a halogen atom (F, Cl, Br, I); and R⁶ denotes a hydrogen atom or a methyl group. Among these, tryptophan, 6-chlorotryptophan, 5-chlorotryptophan and the like are preferable.
   The amino acid residue of formula (10) is represented by: 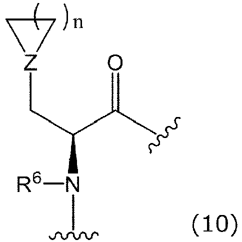 wherein, the wavy line denotes the point of attachment to the carbonyl group or the nitrogen atom that forms the main chain amide bond, Z denotes C or N, and R⁶ denotes a hydrogen atom or a methyl group, and n represents an integer of 1 to 6. Among these, cyclohexylalanine and the like are preferable.
   The amino acid residue of formula (11) is represented by: 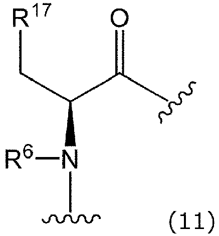 wherein, R¹⁷ denotes a 2-thienyl group, 3-thienyl group, 2-pyridyl group, 3-pyridyl group, 4-pyridyl group, 2-thiazolyl group, 4-thiazolyl group, 2-oxazolyl group, 4-oxazolyl group, 1-pyrazolyl group, 1-imidazolyl group, 1,2,3-triazole-1-yl group, 1,2,4-triazole-1-yl group, 1H-Benzoimidazole-1-yl group, 1H-benzoimidazole-2-yl group, 1,3-benzothiazole-2-yl group, 1,3-benzoxazole-2-yl group, 2-quinolyl group, 8-quinoylyl group, 1-naphthyl group, or 2-naphthyl group, and R⁶ denotes a hydrogen atom or a methyl group. Among these, 2-naphthylalanine and the like are preferable.
[6] X⁶ is an amino acid residue represented by the following formula (12): 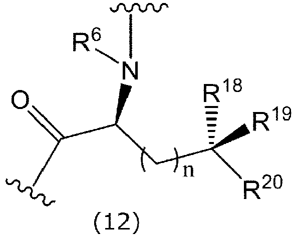 In the formula, the wavy line denotes the point of attachment to the carbonyl group or the nitrogen atom that forms the main chain amide bond; and R¹⁸, R¹⁹, and R²⁰ each independently denotes a hydrogen atom, a hydroxy group, a methoxy group, an amino group, a monomethylated amino group, a dimethylated amino group, a trimethylated amino group, a methyl group, an ethyl group, an isopropyl group, a tert-butyl group, a halogenated methyl group or a halogen atom (F, Cl, Br, I); R⁶ denotes a hydrogen or a methyl group, and n represents 0 or 1. Among these, valine, leucine, serine, threonine and 2, 3-diaminopropanoic acid and the like are preferable.
[7] X² and X⁹, each independently denotes an amino acid residue having a hydrocarbon group optionally having a substituent, N-methyl glycine, 2-azetidine-2-carboxylic acid, proline, thiopurine, 3,4-dehydroproline, pipecolic acid or a derivative thereof.
[8] X¹⁰ denotes valine, norvaline, leucine, norleucine, isoleucine, methionine, cyclopropyl glycine, cyclobutylglycine, phenylalanine, 4-fluoro-phenylalanine, 2-thienylalanine, 3-thienylalanine, 2-pyridylalanine, 3 2-pyridylalanine, 3-pyridylalanine, 4-pyridylalanine, 2-thiazolylalanine, 4-thiazolylalanine, 2-oxazolylalanine, 4-oxazolylalanine, 1-pyrazolylalanine, 1-imidazolylalanine, 3-(1,2,3-triazol-1-yl)-alanine, 3-(1,2,4-triazol-1-yl)-alanine, 3-(1H-benzoimidazol-1-yl)-alanine, 3-(1,3-benzothiazol-2-yl)-alanine, 3-(1,3-benzoxazol-2-yl)-alanine, 2-quinolylalanine, 8-quinolylalanine, tryptophan, 1-naphthylalanine, 2-naphthylalanine, or their N-methylated amino acid residues, or derivatives thereof.
[9] The cyclic peptide in one embodiment of the present invention consists of an amino acid sequence represented by the following formula (13):

   c[X¹-Pro-X³-c(Cys-X⁵-Ser-4fF-Asp-Pro-X¹⁰-X¹¹)] (13)

   In formula (13), X¹ denotes a residue of β-alanine or γ-aminobutyric acid, X³ denotes a residue of leucine, norleucine, cyclohexylglycine, phenylglycine, 2-aminoheptanoic acid, 2-aminooctanoic acid, 2-aminononanonic acid, or 2-aminodecanoic acid, X⁵ denotes a residue of isoleucine, norleucine, cyclohexylglycine, phenylglycine, 2-aminoheptanoic acid, 2-aminooctanoic acid, 2-aminononanonic acid, or 2-aminodecanoic acid, X¹⁰ denotes a residue of valine, phenylalanine, tryptophane or an N-methyl derivative thereof, X¹¹ denotes a residue of D-cysteine or L-cysteine, X¹ and X¹¹ form an amide bond between an amino group and an carboxy group in their main chains, and X⁴ and X¹¹ form a covalent bond between their side chain sulfhydryl groups via a linker of a methylene group, an ethylene group, a propylene group or a butylene group, whereby the peptide of formula (13) has two cyclic structures in the molecule.
[10] The cyclic peptide of a more preferable embodiment consists of an amino acid sequence represented by the following formula (14):

   c[β-Ala-Pro-X³-c(Cys-X⁵-Ser-4fF-Asp-Pro-Trp-^{D}Cys)] (14).

   In formula (14), X³ denotes a residue of leucine, norleucine, cyclohexylglycine, phenylglycine, 2-aminoheptanoic acid, 2-aminooctanoic acid, 2-aminononanonic acid, or 2-aminodecanoic acid, and X⁵ denotes a residue of isoleucine, norleucine, cyclohexylglycine, phenylglycine, 2-aminoheptanoic acid, 2-aminooctanoic acid, 2-aminononanonic acid, or 2-aminodecanoic acid. The residues at position 1 and position 11 form an amide bond between an amino group and a carboxy group in their main chains, and two cysteine resides at position 4 and 11 form a covalent bond between their side chain sulfhydryl groups via a linker of a propylene group, whereby the peptide of formula (14) has two cyclic structures in the molecule.

As for the individual cyclic peptides in formula (13) or (14) above, the following examples can be given:
c[βAla-Pro-Nle-c(Cys-Ile-Ser-4fF-Asp-Pro-Val-Cys)] (SEQ ID NOs:1-3)
c[βAla-Pro-Nle-c(Cys-Ile-Ser-4fF-Asp-Pro-Val-^{D}Cys)] (SEQ ID NOs:4-6)
c[γAba-Pro-Nle-c(Cys-Ile-Ser-4fF-Asp-Pro-Val-Cys)] (SEQ ID NOs:7-9)
c[γAba-Pro-Nle-c(Cys-Ile-Ser-4fF-Asp-Pro-Val-^{D}Cys)] (SEQ ID NOs:10-12)
c[βAla-Pro-Nle-c(Cys-Phg-Ser-4fF-Asp-Pro-Val-^{D}Cys)] (SEQ ID NO:13)
c[βAla-Pro-Nle-c(Cys-Aoc-Ser-4fF-Asp-Pro-Val-^{D}Cys)] (SEQ ID NO:14)
c[βAla-Pro-Nle-c(Cys-Anon-Ser-4fF-Asp-Pro-Val-^{D}Cys)] (SEQ ID NO:15)
c[βAla-Pro-Nle-c(Cys-Anon-Ser-4fF-)Asp-Pro-Phe-^{D}Cys)] (SEQ ID NO:16)
c[βAla-Pro-Nle-c(Cys-Anon-Ser-4fF-Asp-Pro-Trp-^{D}Cys)] (SEQ ID NO:17)
c[βAla-Pro-Nle-c(Cys-Anon-Ser-4fF-Asp-Pro-1NaphA-^{D}Cys)] (SEQ ID NO:18)
c[pAla-Pro-Anon-c(Cys-Anon-Ser-4fF-Asp-Pro-Trp-^{D}Cys)] (SEQ ID NO: 19)
c[pAla-Pro-Nle-c(Cys-Aoc-Ser-4fF-Asp-Pro-^{m}Val-^{D}Cys)] (SEQ ID NOs: 20-21)

c[βAla-Pro-Leu-c(Cys-Ile-Ser-4fF-Asp-Pro-Trp-^{D}Cys)]
c[βAla-Pro-Leu-c(Cys-Nle-Ser-4fF-Asp-Pro-Trp-^{D}Cys)]
c[βAla-Pro-Leu-c(Cys-Chg-Ser-4fF-Asp-Pro-Trp-^{D}Cys)]
c[βAla-Pro-Leu-c(Cys-Phg-Ser-4fF-Asp-Pro-Trp-^{D}Cys)]
c[βAla-Pro-Leu-c(Cys-Ahep-Ser-4fF-Asp-Pro-Trp-^{D}Cys)]
c[βAla-Pro-Leu-c(Cys-Aoc-Ser-4fF-Asp-Pro-Trp-^{D}Cys)]
c[βAla-Pro-Leu-c(Cys-Anon-Ser-4fF-Asp-Pro-Trp-^{D}Cys)]
c[βAla-Pro-Leu-c(Cys-Adec-Ser-4fF-Asp-Pro-Trp-^{D}Cys)]
c[βAla-Pro-Nle-c(Cys-Ile-Ser-4fF-Asp-Pro-Trp-^{D}Cys)]
c[βAla-Pro-Nle-c(Cys-Nle-Ser-4fF-Asp-Pro-Trp-^{D}Cys)]
c[βAla-Pro-Nle-c(Cys-Chg-Ser-4fF-Asp-Pro-Trp-^{D}Cys)]
c[βAla-Pro-Nle-c(Cys-Phg-Ser-4fF-Asp-Pro-Trp-^{D}Cys)]
c[βAla-Pro-Nle-c(Cys-Ahep-Ser-4fF-Asp-Pro-Trp-^{D}Cys)]
c[βAla-Pro-Nle-c(Cys-Aoc-Ser-4fF-Asp-Pro-Trp-^{D}Cys)]
c[βAla-Pro-Nle-c(Cys-Adec-Ser-4fF-Asp-Pro-Trp-^{D}Cys)]
c[βAla-Pro-Chg-c(Cys-Ile-Ser-4fF-Asp-Pro-Trp-^{D}Cys)]
c[βAla-Pro-Chg-c(Cys-Nle-Ser-4fF-Asp-Pro-Trp-^{D}Cys)]
c[βAla-Pro-Chg-c(Cys-Chg-Ser-4fF-Asp-Pro-Trp-^{D}Cys)]
c[βAla-Pro-Chg-c(Cys-Phg-Ser-4fF-Asp-Pro-Trp-^{D}Cys)]
c[βAla-Pro-Chg-c(Cys-Ahep-Ser-4fF-Asp-Pro-Trp-^{D}Cys)]
c[βAla-Pro-Chg-c(Cys-Aoc-Ser-4fF-Asp-Pro-Trp-^{D}Cys)]
c[βAla-Pro-Chg-c(Cys-Anon-Ser-4fF-Asp-Pro-Trp-^{D}Cys)]
c[βAla-Pro-Chg-c(Cys-Adec-Ser-4fF-Asp-Pro-Trp-^{D}Cys)]
c[βAla-Pro-Phg-c(Cys-Ile-Ser-4fF-Asp-Pro-Trp-^{D}Cys)]
c[βAla-Pro-Phg-c(Cys-Nle-Ser-4fF-Asp-Pro-Trp-^{D}Cys)]
c[βAla-Pro-Phg-c(Cys-Chg-Ser-4fF-Asp-Pro-Trp-^{D}Cys)]
c[βAla-Pro-Phg-c(Cys-Phg-Ser-4fF-Asp-Pro-Trp-^{D}Cys)]
c[βAla-Pro-Phg-c(Cys-Ahep-Ser-4fF-Asp-Pro-Trp-^{D}Cys)]
c[βAla-Pro-Phg-c(Cys-Aoc-Ser-4fF-Asp-Pro-Trp-^{D}Cys)]
c[βAla-Pro-Phg-c(Cys-Anon-Ser-4fF-Asp-Pro-Trp-^{D}Cys)]
c[βAla-Pro-Phg-c(Cys-Adec-Ser-4fF-Asp-Pro-Trp-^{D}Cys)]
c[βAla-Pro-Ahep-c(Cys-Ile-Ser-4fF-Asp-Pro-Trp-^{D}Cys)]
c[βAla-Pro-Ahep-c(Cys-Nle-Ser-4fF-Asp-Pro-Trp-^{D}Cys)]
c[βAla-Pro-Ahep-c(Cys-Chg-Ser-4fF-Asp-Pro-Trp-^{D}Cys)]
c[βAla-Pro-Ahep-c(Cys-Phg-Ser-4fF-Asp-Pro-Trp-^{D}Cys)]
c[βAla-Pro-Ahep-c(Cys-Ahep-Ser-4fF-Asp-Pro-Trp-^{D}Cys)]
c[βAla-Pro-Ahep-c(Cys-Aoc-Ser-4fF-Asp-Pro-Trp-^{D}Cys)]
c[βAla-Pro-Ahep-c(Cys-Anon-Ser-4fF-Asp-Pro-Trp-^{D}Cys)]
c[βAla-Pro-Ahep-c(Cys-Adec-Ser-4fF-Asp-Pro-Trp-^{D}Cys)]
c[βAla-Pro-Aoc-c(Cys-Ile-Ser-4fF-Asp-Pro-Trp-^{D}Cys)]
c[βAla-Pro-Aoc-c(Cys-Nle-Ser-4fF-Asp-Pro-Trp-^{D}Cys)]
c[βAla-Pro-Aoc-c(Cys-Chg-Ser-4fF-Asp-Pro-Trp-^{D}Cys)]
c[βAla-Pro-Aoc-c(Cys-Phg-Ser-4fF-Asp-Pro-Trp-^{D}Cys)]
c[βAla-Pro-Aoc-c(Cys-Ahep-Ser-4fF-Asp-Pro-Trp-^{D}Cys)]
c[βAla-Pro-Aoc-c(Cys-Aoc-Ser-4fF-Asp-Pro-Trp-^{D}Cys)]
c[βAla-Pro-Aoc-c(Cys-Anon-Ser-4fF-Asp-Pro-Trp-^{D}Cys)]
c[βAla-Pro-Aoc-c(Cys-Adec-Ser-4fF-Asp-Pro-Trp-^{D}Cys)]
c[βAla-Pro-Anon-c(Cys-Ile-Ser-4fF-Asp-Pro-Trp-^{D}Cys)]
c[βAla-Pro-Anon-c(Cys-Nle-Ser-4fF-Asp-Pro-Trp-^{D}Cys)]
c[βAla-Pro-Anon-c(Cys-Chg-Ser-4fF-Asp-Pro-Trp-^{D}Cys)]
c[βAla-Pro-Anon-c(Cys-Phg-Ser-4fF-Asp-Pro-Trp-^{D}Cys)]
c[βAla-Pro-Anon-c(Cys-Ahep-Ser-4fF-Asp-Pro-Trp-^{D}Cys)]
c[βAla-Pro-Anon-c(Cys-Aoc-Ser-4fF-Asp-Pro-Trp-^{D}Cys)]
c[βAla-Pro-Anon-c(Cys-Adec-Ser-4fF-Asp-Pro-Trp-^{D}Cys)]
c[βAla-Pro-Adec-c(Cys-Ile-Ser-4fF-Asp-Pro-Trp-^{D}Cys)]
c[βAla-Pro-Adec-c(Cys-Nle-Ser-4fF-Asp-Pro-Trp-^{D}Cys)]
c[βAla-Pro-Adec-c(Cys-Chg-Ser-4fF-Asp-Pro-Trp-^{D}Cys)]
c[βAla-Pro-Adec-c(Cys-Phg-Ser-4fF-Asp-Pro-Trp-^{D}Cys)]
c[βAla-Pro-Adec-c(Cys-Ahep-Ser-4fF-Asp-Pro-Trp-^{D}Cys)]
c[βAla-Pro-Adec-c(Cys-Aoc-Ser-4fF-Asp-Pro-Trp-^{D}Cys)]
c[βAla-Pro-Adec-c(Cys-Anon-Ser-4fF-Asp-Pro-Trp-^{D}Cys)]
c[βAla-Pro-Adec-c(Cys-Adec-Ser-4fF-Asp-Pro-Trp-^{D}Cys)]

c[βAla-Pro-Leu-c(Cys-Ile-Ser-4fF-Asp-Pro-Trp-Cys)]
c[βAla-Pro-Leu-c(Cys-Nle-Ser-4fF-Asp-Pro-Trp-Cys)]
c[βAla-Pro-Leu-c(Cys-Chg-Ser-4fF-Asp-Pro-Trp-Cys)]
c[βAla-Pro-Leu-c(Cys-Phg-Ser-4fF-Asp-Pro-Trp-Cys)]
c[βAla-Pro-Leu-c(Cys-Ahep-Ser-4fF-Asp-Pro-Trp-Cys)]
c[βAla-Pro-Leu-c(Cys-Aoc-Ser-4fF-Asp-Pro-Trp-Cys)]
c[βAla-Pro-Leu-c(Cys-Anon-Ser-4fF-Asp-Pro-Trp-Cys)]
c[βAla-Pro-Leu-c(Cys-Adec-Ser-4fF-Asp-Pro-Trp-Cys)]
c[βAla-Pro-Nle-c(Cys-Ile-Ser-4fF-Asp-Pro-Trp-Cys)]
c[βAla-Pro-Nle-c(Cys-Nle-Ser-4fF-Asp-Pro-Trp-Cys)]
c[βAla-Pro-Nle-c(Cys-Chg-Ser-4fF-Asp-Pro-Trp-Cys)]
c[βAla-Pro-Nle-c(Cys-Phg-Ser-4fF-Asp-Pro-Trp-Cys)]
c[βAla-Pro-Nle-c(Cys-Ahep-Ser-4fF-Asp-Pro-Trp-Cys)]
c[βAla-Pro-Nle-c(Cys-Aoc-Ser-4fF-Asp-Pro-Trp-Cys)]
c[βAla-Pro-Nle-c(Cys-Anon-Ser-4fF-Asp-Pro-Trp-Cys)]
c[βAla-Pro-Nle-c(Cys-Adec-Ser-4fF-Asp-Pro-Trp-Cys)]
c[βAla-Pro-Chg-c(Cys-Ile-Ser-4fF-Asp-Pro-Trp-Cys)]
c[βAla-Pro-Chg-c(Cys-Nle-Ser-4fF-Asp-Pro-Trp-Cys)]
c[βAla-Pro-Chg-c(Cys-Chg-Ser-4fF-Asp-Pro-Trp-Cys)]
c[βAla-Pro-Chg-c(Cys-Phg-Ser-4fF-Asp-Pro-Trp-Cys)]
c[βAla-Pro-Chg-c(Cys-Ahep-Ser-4fF-Asp-Pro-Trp-Cys)]
c[βAla-Pro-Chg-c(Cys-Aoc-Ser-4fF-Asp-Pro-Trp-Cys)]
c[βAla-Pro-Chg-c(Cys-Anon-Ser-4fF-Asp-Pro-Trp-Cys)]
c[βAla-Pro-Chg-c(Cys-Adec-Ser-4fF-Asp-Pro-Trp-Cys)]
c[βAla-Pro-Phg-c(Cys-Ile-Ser-4fF-Asp-Pro-Trp-Cys)]
c[βAla-Pro-Phg-c(Cys-Nle-Ser-4fF-Asp-Pro-Trp-Cys)]
c[βAla-Pro-Phg-c(Cys-Chg-Ser-4fF-Asp-Pro-Trp-Cys)]
c[βAla-Pro-Phg-c(Cys-Phg-Ser-4fF-Asp-Pro-Trp-Cys)]
c[βAla-Pro-Phg-c(Cys-Ahep-Ser-4fF-Asp-Pro-Trp-Cys)]
c[βAla-Pro-Phg-c(Cys-Aoc-Ser-4fF-Asp-Pro-Trp-Cys)]
c[βAla-Pro-Phg-c(Cys-Anon-Ser-4fF-Asp-Pro-Trp-Cys)]
c[βAla-Pro-Phg-c(Cys-Adec-Ser-4fF-Asp-Pro-Trp-Cys)]
c[βAla-Pro-Ahep-c(Cys-Ile-Ser-4fF-Asp-Pro-Trp-Cys)]
c[βAla-Pro-Ahep-c(Cys-Nle-Ser-4fF-Asp-Pro-Trp-Cys)]
c[βAla-Pro-Ahep-c(Cys-Chg-Ser-4fF-Asp-Pro-Trp-Cys)]
c[βAla-Pro-Ahep-c(Cys-Phg-Ser-4fF-Asp-Pro-Trp-Cys)]
c[βAla-Pro-Ahep-c(Cys-Ahep-Ser-4fF-Asp-Pro-Trp-Cys)]
c[βAla-Pro-Ahep-c(Cys-Aoc-Ser-4fF-Asp-Pro-Trp-Cys)]
c[βAla-Pro-Ahep-c(Cys-Anon-Ser-4fF-Asp-Pro-Trp-Cys)]
c[βAla-Pro-Ahep-c(Cys-Adec-Ser-4fF-Asp-Pro-Trp-Cys)]
c[βAla-Pro-Aoc-c(Cys-Ile-Ser-4fF-Asp-Pro-Trp-Cys)]
c[βAla-Pro-Aoc-c(Cys-Nle-Ser-4fF-Asp-Pro-Trp-Cys)]
c[βAla-Pro-Aoc-c(Cys-Chg-Ser-4fF-Asp-Pro-Trp-Cys)]
c[βAla-Pro-Aoc-c(Cys-Phg-Ser-4fF-Asp-Pro-Trp-Cys)]
c[βAla-Pro-Aoc-c(Cys-Ahep-Ser-4fF-Asp-Pro-Trp-Cys)]
c[βAla-Pro-Aoc-c(Cys-Aoc-Ser-4fF-Asp-Pro-Trp-Cys)]
c[βAla-Pro-Aoc-c(Cys-Anon-Ser-4fF-Asp-Pro-Trp-Cys)]
c[βAla-Pro-Aoc-c(Cys-Adec-Ser-4fF-Asp-Pro-Trp-Cys)]
c[βAla-Pro-Anon-c(Cys-Ile-Ser-4fF-Asp-Pro-Trp-Cys)]
c[βAla-Pro-Anon-c(Cys-Nle-Ser-4fF-Asp-Pro-Trp-Cys)]
c[βAla-Pro-Anon-c(Cys-Chg-Ser-4fF-Asp-Pro-Trp-Cys)]
c[βAla-Pro-Anon-c(Cys-Phg-Ser-4fF-Asp-Pro-Trp-Cys)]
c[βAla-Pro-Anon-c(Cys-Ahep-Ser-4fF-Asp-Pro-Trp-Cys)]
c[βAla-Pro-Anon-c(Cys-Aoc-Ser-4fF-Asp-Pro-Trp-Cys)]
c[βAla-Pro-Anon-c(Cys-Anon-Ser-4fF-Asp-Pro-Trp-Cys)]
c[βAla-Pro-Anon-c(Cys-Adec-Ser-4fF-Asp-Pro-Trp-Cys)]
c[βAla-Pro-Adec-c(Cys-Ile-Ser-4fF-Asp-Pro-Trp-Cys)]
c[βAla-Pro-Adec-c(Cys-Nle-Ser-4fF-Asp-Pro-Trp-Cys)]
c[βAla-Pro-Adec-c(Cys-Chg-Ser-4fF-Asp-Pro-Trp-Cys)]
c[βAla-Pro-Adec-c(Cys-Phg-Ser-4fF-Asp-Pro-Trp-Cys)]
c[βAla-Pro-Adec-c(Cys-Ahep-Ser-4fF-Asp-Pro-Trp-Cys)]
c[βAla-Pro-Adec-c(Cys-Aoc-Ser-4fF-Asp-Pro-Trp-Cys)]
c[βAla-Pro-Adec-c(Cys-Anon-Ser-4fF-Asp-Pro-Trp-Cys)]
c[βAla-Pro-Adec-c(Cys-Adec-Ser-4fF-Asp-Pro-Trp-Cys)]

[11] The peptides of the present invention include homologous peptides in which one or several amino acids are deleted, added, and/or substituted in the amino acid sequences represented in [1] through [10] above, provided that they have binding activity against Ras. When the term "peptide in which one to several amino acids are deleted, added, and/or substituted" is used herein, the number of those amino acids is not particularly limited as long as the peptide has Ras-binding activity, but is preferably one to five, and even more preferably one or two. Positions of the deletion, addition, and/or substitution may be at the end or in the middle of the peptide sequence, and may be in one or more locations.

In one aspect, as an amino acid sequence in which one or several amino acids are deleted, added, and/or substituted in the above amino acid sequence, such an amino acid sequence is exemplified as those having at least 50%, preferably 70%, even more preferably 80%, and especially preferably 90% or more identity with said amino acid sequence, upon calculation using BLAST (Basic Local Alignment Search Tool at the National Center for Biological Information) or the like (e.g., using default i.e., default parameters).

It is also known that there are peptides and protein domains with highly similar three-dimensional structures, even if the homology in the primary structure is low. Therefore, peptides having at least 50%, preferably 70%, even more preferably 80%, and especially preferably 90% or more three-dimensional structure homology with the above amino acid sequence and having Ras binding activity are also included in this embodiment. The homology of the three-dimensional structures of peptides can be predicted as follows from the amino acid sequence of a peptide having an unknown three-dimensional structure by using a homology modeling method or the like. For example, when an arbitrary amino acid sequence (target sequence) having a sequence similar to the cyclic peptide (reference peptide) of the present embodiment is given, an alignment (sequence juxtaposition) between the target sequence and the reference sequence is given. Using the alignment calculated by FASTA, PSI-BLAST, LIBRA, etc., the correspondence relationship for each amino acid between the target sequence and the reference sequence can be determined, and based on this relationship, the 3D coordinates for each amino acid on the target sequence are created from the 3D coordinates of the reference peptide. In the construction of the 3D coordinates, there may be structurally inappropriate gaps, collisions, or distortions between amino acid residues, so these structural distortions are resolved by energy minimization calculations. In some modeling software, the structural distortions are resolved in stages rather than simultaneously for all atoms of the peptide. In other words, the alpha carbon atoms that form the backbone of the peptide are first processed, followed by the main chain atoms that contain the alpha carbon atoms, and finally the entire peptide including the side chain atoms. Once the alignment to the target sequence is obtained in this way, the 3D structure can be predicted and constructed. The 3D structure homology index can be compared using RMSD (Root Mean Square Deviation), which is the difference between the XYZ coordinates when they are optimally superimposed.

The peptides of the present invention also encompass various derivatives and/or modifications thereof, as long as they solve the problems of the present invention. Such derivatives include but are not limited to those in which the saturated fatty chain of the peptide is replaced with an unsaturated fatty chain; some of the atoms of the peptide are replaced with other atoms containing radioactive or non-radioactive isotope atoms; the amide bond of the peptide is replaced with a thioamide bond (-NH-C(=S)-); the amide bond of the peptide is replaced with alken (-C=C-) ; the amide bond of the peptide is substituted with alkyl (-C-C-) ; the amide bond of the peptide is substituted with hydroxyethylene (-C(-OH)-C-); the amide bond of the peptide is replaced with ester (-O-C(=O)-); the amide bond of the peptide is substituted with (-C-NH-) ; or the amide bond of the peptide is replaced with (-C(=O)-C-). The above modifications include but are not limited to those in which the α-carbon of the peptide is disubstituted; the amide bond of the peptide is N-alkylated; some of the functional groups of the peptide are halogenation, cyanation, nitration, hydroformylation, hydroxylation, amination, deamination, dehydroxylation, amidation, acetylation, methoxylation, prenylation, alkylation, and the like (e.g., some of the amino groups of the peptides are acetylated, alkylated, or deaminated, some of the carboxy groups of the peptides are amide or ester, etc.); S of the peptide is sulfoxide S(=O) or sulfone S(=O)₂; the peptide is multimerized via chemical linkers; the peptide is biotin-labeled; the peptide is fluorescently labeled; the peptide is fluorescently labeled; further, the peptide fused to alkyl chains, polyethylene glycols, antibodies, lectins, glycans, enzymes, membrane-permeable peptides, low molecular weight compounds, or molecules that induce ubiquitination of proteins.

The peptides of the present invention also encompass salts of the peptide. The salts of the peptides can be salts with physiologically acceptable bases or acids, and for example, an addition salt of an inorganic acid (hydrochloric acid, hydrobromic acid, hydroiodic acid, sulfuric acid, phosphoric acid, and the like), addition salts of organic acids (p-toluene sulfonic acid, methane sulfonic acid, oxalic acid, p-bromophenyl sulfonic acid, carboxylic acid, succinic acid, citric acid, benzoic acid, acetic acid, and the like), an addition salt of inorganic bases (ammonium hydroxide or alkali , alkaline earth metal hydroxides, carbonates, bicarbonates, and the like), an addition salt of amino acids or the like.

The peptides of the present invention may also be prodrugs. A prodrug is a compound that is converted to the peptide of the present invention by an enzymatic reaction or gastric acid under physiological conditions *in vivo,* i.e. a compound that is enzymatically oxidized, reduced, or hydrolyzed to the peptide of the present invention, or a compound that is hydrolyzed by gastric acid or the like to the peptide of the present invention.

Examples of the prodrug of the peptide of the present invention include but are not limited to compounds in which the amino group of the peptide of the present invention is acylated, alkylated or phosphorylated (for example, compounds in which the amino group of the peptide of the present invention is eicosanoylated, alanylated, pentylaminocarbonylated, (5-methyl-2-methyl-1,3-dioxolen-4-yl) methoxycarbonylated, tetrahydrofuranylated, pyrrolidylmethylated, pivaloyloxymethylated (5-methyl-2-oxo-1,3-dioxolen-4-yl) methoxycarbonylated, tetrahydrofuranylated, pyrrolidylmethylated, pivaloyloxymethylated, or tert-butylated; compounds in which the hydroxy group of the peptide of the invention is acylated, alkylated, phosphorylated, or borylated (e.g., compounds in which the hydroxy group of the peptide of the invention is acetylated, palmitoylated, propanoylated, pivaloylated, succinylated, fumarylated, alanylated, or dimethylaminomethylcarbonylated) ; compounds in which the hydroxy or carboxy groups of the peptides of the present invention are esterified or amidized (e.g., compounds in which the hydroxy or carboxy groups of the peptides of the present invention are C₁₋₆ alkyl esterified, phenyl esterified, carboxymethyl esterified dimethylaminomethyl esterified, pivaloyloxy methyl esterified, ethoxycarbonyloxy ethyl esterified, phthalidyl esterified, (5-methyl-2-oxo-1,3 -dioxolen-4-yl) methylesterified, cyclohexyloxycarbonylethyl esterified, or methylamidated). These compounds can be produced from the peptides of the present invention by methods known to themselves.

Prodrugs of the peptides of the invention may be those that change to the peptides of the invention under physiological conditions as described in "Development of Medicines" published by Hirokawa Shoten 1990, Volume 7, Molecular Design, pages 163-198.

In this specification, the prodrugs may form salts, and such salts include those exemplified as salts of the peptides of the present invention.

The peptides of the present invention may be crystals, and encompass both a single crystalline form and a mixture of crystalline forms. Crystals can be produced by crystallization, applying crystallization methods known *per se.*

The peptides of the present invention may be pharmaceutically acceptable co-crystals or co-crystal salts. Co-crystals or co-crystal salts are crystalline substances comprising two or more unique solids at room temperature, each having different physical properties (e.g., structure, melting point, heat of fusion, hygroscopicity, solubility, and stability). Co-crystals, or co-crystal salts, can be produced according to co-crystallization methods known per se.

### (Action and Effect of Cyclic Peptide)

As shown in Examples described later, Seq-17, Seq-20 (LO2) and Seq-21 (LO3), which are representative examples in the amino acid sequence group of this embodiment, are resistant to protease degradation, and have growth inhibition activity against K-Ras(G12D)-expressing cells. Since each of the peptides included in the amino acid sequence group of this embodiment has a similar amino acid sequence and a three-dimensional structural feature to these representative examples, they are also very likely to have resistance to protease degradation and cell growth inhibitory activity.

KRpep-2d binds not only to K-Ras (G12D) but also to K-Ras (G12C) and wild-type K-Ras, and it has been confirmed that the binding activity of KRpep-2d to GDP-binding Ras is almost equivalent to that of GTP-binding Ras (Non-Patent Literature 3 and Non-Patent Literature 4). And the amino acid sequence of Ras is highly conserved regardless of the presence or absence of mutation. As shown in Examples described later, the biotin modified peptide (Biotin-BC-1) of the representative example of the amino acid sequence group of this embodiment binds to wild-type K-Ras, K-Ras (G12D), K- Ras (G12V), K-Ras (G12C), K-Ras (G13D), wild type N-Ras, N-Ras (Q61K), wild type H-Ras, H-Ras (G12V), and H-Ras (Q61L) . Since each peptide in the amino acid sequence group of this embodiment has a similar amino acid sequence and a three-dimensional structural feature to those of KRpep-2d pharmacophore and Biotin-BC-1, it is highly likely to bind to all Ras such as mutant N-Ras and mutant H-Ras, and to have a binding activity for both GDP-bound Ras and GTP-bound Ras, thereby inhibiting the function of Ras in general.

K-Ras (G12D) inhibitory activity of KRpep-2d modified peptide described in Non-Patent Literature 5, and several disclosures shown in Examples described later, such as, K-Ras(G12D) binding activity of 2d-5/15-Dap/Asp, K-Ras (G12D) binding activity of Biotin-BC-1, and growth inhibitory activities to K-Ras(G12D)-expressing cells of Seq-1 to Seq-21 show the following. In the bicyclic peptides of the present invention, (1) the covalent bond between positions 1 and 11 can accept a wide range of structural shapes, from flexible structures such as a S-S bond to relatively rigid structures with planarity such as an amide bond. (2) the covalent bond between positions 1 and 11 accepts a wide range of distances from 3 to 7 atoms between the Cα carbons, and (3) the covalent bond between positions 4 and 11 accepts a wide range of distances from 4 to 8 atoms between the Cα carbons.

As shown in Non-Patent Literature 5, since the target-binding activity of cyclic peptides generally follows a bell-shape pattern with the optimum ring size at the apex, the covalent bond between positions 4 and 11 of the bicyclic peptide of the present invention is extremely likely to be accepted if the number of atoms between the Cα carbons is increased by another one or two atoms, with 4-8 at the apex. In other words, the covalent bond between positions 4 and 11 is considered to accept a wide range of distances from 4 to 10 atoms between the Cα carbons. The covalent bond between positions 4 and 11 contributes to the regulation of the bicyclic structure of the peptide, but does not contribute directly to the interaction with Ras. Thus, the covalent bond between positions 4 and 11 is expected to accept a wide range of shape structures, including an amide bond, a disulfide bond, a thioether bond, a C=C bond, a C-C bond, a bond through triazole, and a bond through dithio-tetrafluorobenzene.

Since the amino acid sequence of Ras in general is highly conserved in mammals, it is very likely that a group of peptides comprising the amino acid sequence represented in this embodiment will bind to and inhibit the function of mutant K-Ras, mutant N-Ras, and/or mutant H-Ras in non-human mammals such as mice, rats, dogs, and monkeys.

### (Method for producing cyclic peptide)

The peptides in this embodiment can be produced by known peptide production methods, such as chemical synthesis methods, including liquid-phase methods, solid-phase methods, or hybrid methods that combine liquid-phase and solid-phase methods.

For the solid phase method, commercially available automated synthesizers can be used. For example, an esterification reaction is performed between the hydroxyl group of a resin having a hydroxyl group and the carboxyl group of a primary amino acid (usually the C-terminal amino acid of the peptide of interest) whose α-amino group is protected by a protecting group such as the Fmoc group. As an esterification catalyst, known dehydration and condensation agents such as 1-mesitylenesulfonyl-3-nitro-1,2,4-triazole (MSNT), dicyclohexylcarbodiimide (DCC), and diisopropylcarbodiimide (DIPCDI) can be used. Next, the protecting group of the α-amino group of the first amino acid is deprotected and a second amino acid, in which all functional groups except the carboxy group of the main chain are protected, is added to activate the carboxy group and bind the first and second amino acids together. Furthermore, the α-amino group of the second amino acid is deprotected, and a third amino acid, in which all functional groups except the carboxy group of the main chain are protected, is added to activate the carboxy group, and the second and third amino acids are combined. This process is repeated to synthesize a peptide of the desired length. The linear peptide is cleaved from the resin, purified, and the functional group for cyclizing the peptide is deprotected, according to the standard method.

Solid-phase synthesis resins include Merrifield resin, MBHA resin, Cl-Trt resin, SASRIN resin, Wang resin, Rink amide resin, HMFS resin, Amino-PEGA resin (Merck), and HMPA-PEGA resin (Merck) and the like. These resins may be washed with a solvent (dimethylformamide (DMF), 2-propanol, methylene chloride, etc.) before use. The protective group for the α-amino group includes a benzyloxycarbonyl (Cbz) group, a tert-butoxycarbonyl (Boc) group, a fluorenylmethoxycarbonyl (Fmoc) group, a benzyl group, an allyl group, and an allyloxycarbonyl (Allloc) group and the like. The Cbz group can be deprotected by hydrofluoric acid, hydrogenation, etc. The Boc group can be deprotected by trifluoroacetic acid (TFA), and the Fmoc group can be deprotected by treatment with piperidine. The α-carboxy group can be protected by methyl ester, ethyl ester, benzyl ester, tert-butyl ester, cyclohexyl ester, etc. Other functional groups of amino acids, such as hydroxy groups of serine and threonine can be protected by benzyl or tert-butyl groups, and hydroxy groups of tyrosine can be protected by 2-bromobenzyloxycarbonyl or tert-butyl groups. Side chain amino groups such as lysine, and carboxy groups such as glutamic acid and aspartic acid can be protected in the same way as alpha-amino groups and alpha-carboxy groups.

Activation of the carboxy group can be carried out using a condensing agent. Examples of condensing agents are dicyclohexylcarbodiimide (DCC), diisopropylcarbodiimide (DIPCDI), 1-ethyl-3-(3-dimethylaminopropyl) carbodiimide (EDC or WSC), (1H-benzotriazol-1-yloxy)tris(dimethylamino)phosphonium hexafluorophosphate (BOP) bis(dimethylamino)methyl]-1H-benzotriazolium-3-oxide hexafluorophosphate (HBTU), and the like. Cleavage of the peptide chain from the resin can be carried out by treatment with acids such as TFA and hydrogen fluoride (HF).

The peptide of this embodiment is cyclized as one aspect. In the present specification, cyclization means the covalent linkage of two or more amino acids separated by one or more amino acids within a peptide, either directly or indirectly via a linker, to create one or more cyclic structures within the molecule. Cyclization can be carried out in accordance with the methods described in non-patent Literatures 3, 6 and 7. Non-limiting examples of cyclization are amide bond between amino group and carboxy group, disulfide bond between thiol group and thiol group, thioether bond between thiol group and halogen group, thioether bond between thiol group and allyl group by thiol-ene reaction, C=C bond by an olefin metathesis reaction between an allyl group and an allyl group (the C=C bond may be converted to a C-C bond by a reduction reaction), a triazole-mediated bond by a click reaction between an alkynyl group and an azide group, a thioether bond between a linker having a halogen group and two thiol groups, and the like. The direct or indirect covalent bond for cyclization may be main-chain-to-main-chain, main-chain-to-side chain, side-chain-to-main-chain, or side-chain-to-side chain.

For example, the following bonds (linkages) can be used for the cyclization of the peptides of the present invention: (1) a disulfide bond formed between the thiol groups of cysteine, D-cysteine, homocysteine, and D-homocysteine with thiol groups as amino acids 1 and 2, respectively; (2) a thioether bond formed between amino acid 1 having a nucleophilic halogen atom (chloro, bromo, or iodo) (e.g., 3-chloroalanine), or a carboxylic acid with a halogen atom (chloro, bromo, or iodo) (e.g., 3-chloropropanoic acid), and amino acid 2 having a thiol group; (3) a thioether bond formed between amino acids 1 and 2 having a thiol group and a linker having a halogen atom (chloro, bromo, or iodo) as a nucleophile (e.g., diiodomethane (DIM), 1,2-diiodoethane (DIE), 1,3-diiodopropane (DIP), 1,4-diiodobutane (DIB), hexafluorobenzene, etc.); (4) a covalent bond via triazole formed by click reaction between β-azidoalanine with azide group as amino acid 1 and 2-amino-5-hexinic acid with alkynyl group as amino acid 2; (5) a thioether bond formed by a thiol-ene reaction between an amino acid with an allyl group (e.g., allylglycine, D-allylglycine, homoallylglycine, D-homoallylglycine, etc.) or a carboxylic acid with an allyl group (e.g., 3-butenoic acid) as amino acid 1 and amino acid 2 with a thiol group; (6) a C=C bond formed by olefin metathesis reaction between amino acids with allyl groups or carboxylic acids with allyl groups as amino acids 1 and 2, respectively, and their allyl groups; (7) a C-C bond formed by reduction of the C=C bond formed by olefin metathesis reaction; (8) an amide bond between amino acid 1 having an amino group (e.g. 2,3-diaminopropanoic acid, 2,4-diaminobutanoic acid, ornithine, lysine, their D-amino acids, beta-alanine, etc.) and amino acid 2 having a carboxy group (e.g. aspartic acid, glutamic acid, their D-amino acids, etc.) or C-terminal carboxy group; (9) an amide bond between an N-terminal amino group and an amino acid with a carboxy group or a C-terminal carboxy group. Either amino acid 1 or amino acid 2 may come to the N-terminal side.

### (Pharmaceuticals, diagnostics, and research reagents containing cyclic peptides)

The pharmaceutical composition of the present invention contains a peptide comprising the amino acid sequence described above as an active ingredient and can inhibit the growth of malignant tumors and the like through binding of the peptide to Ras and inhibition of its activity. The dosage form of the above pharmaceutical composition is not particularly limited and may be administered orally or parenterally. Parenteral administration includes, for example, injection administration such as intramuscular, intravenous, and subcutaneous injections, transdermal administration, and transmucosal administration (nasal, transoral, transocular, transpulmonary, transvaginal, or transrectal administration). The peptides in the pharmaceutical compositions can be modified in various ways in view of their propensity to be metabolized and excreted. For example, alkyl chains, polyethylene glycols, or sugar chains can be added to the peptide to increase its retention time in the bloodstream and reduce its antigenicity. In addition, bio-degradable polymeric compounds such as polylactic acid and glycol (PLGA), porous hydroxyapatite, liposomes, surface-modified liposomes, emulsions prepared with unsaturated fatty acids, nanoparticles, nanospheres, etc. can be used as sustained release base agents, and the peptides can be encapsulated in them. When administered transdermally, a weak electric current can also be applied to the skin surface to penetrate the stratum corneum (iontophoresis method).

The above pharmaceutical compositions may be used as the active ingredients as they are, or they may be formulated by adding pharmaceutically acceptable carriers, excipients, additives, etc. Examples of dosage forms include liquids (e.g., injections), dispersants, suspensions, tablets, rounds, powders, suppositories, sprays, fine granules, granules, capsules, syrups, lozenges, inhalants, ointments, eye drops, nasal drops, ear drops, and poultice preparations. These formulations may be controlled release formulations (e.g., sustained release microcapsules), such as fast or sustained release formulations. Formulation can be carried out according to conventional methods using, for example, excipients, binders, disintegrants, lubricants, dissolving agents, dissolution aids, coloring agents, taste and odor correcting agents, stabilizers, emulsifiers, absorption enhancers, surfactants, pH adjusters, preservatives, antioxidants, and the like. Examples of ingredients used in formulation include but are not limited to purified water, saline solution, phosphate buffer solution, dextrose, glycerol, ethanol and other pharmaceutically acceptable organic solvents, animal and vegetable oils, lactose, mannitol, glucose, sorbitol, crystal cellulose, hydroxypropyl cellulose, starch, corn starch, silicic acid, aluminum silicate magnesium, collagen, polyvinyl alcohol, poliovinylpyrrolidone, carboxyvinyl polymer, sodium carboxymethyl cellulose, sodium polyacrylate, sodium alginate, water-soluble dextran, sodium carboxymethyl starch, pectin methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, human serum albumin, and the like. If peptides are not readily absorbed by the transmucosal membrane, absorption enhancers that improve the absorption of poorly absorbed drugs include surfactants such as polyoxyethylene lauryl ethers, sodium lauryl sulfate, and saponin; bile salts such as glycocholic acid, deoxycholic acid, and taurocholic acid; chelating agents such as EDTA and salicylic acid; and caproic acid. Chelating agents; fatty acids such as caproic acid, capric acid, lauric acid, oleic acid, linoleic acid, mixed micelles, etc.; enamine derivatives, N-acyl collagen peptides, N-acylamino acids, cyclodextrins, chitosans, nitric oxide donors, and the like may also be used.

Round or tablet formulations can also be coated with sugar-coated, gastric- or enteric-soluble substances. Injectable formulations can contain distilled water for injection, saline, propylene glycol, polyethylene glycol, vegetable oils, alcohols, etc. In addition, wetting agents, emulsifying agents, dispersing agents, stabilizing agents, dissolving agents, dissolution aids, and preservatives can be added. If necessary, additives such as ordinary preservatives, antioxidants, coloring agents, sweetening agents, adsorbents, wetting agents, etc. can also be used in appropriate amounts.

The pharmaceutical compositions of the present invention are effective in the treatment or prevention of various diseases believed to be caused by malignant tumors expressing Ras by inhibiting the function of Ras. Examples include, but are not limited to, pancreatic cancer (e.g., pancreatic ductal carcinoma, etc.), colorectal cancer (e.g., familial colorectal cancer, hereditary nonpolyposis colorectal cancer, gastrointestinal stromal tumor, etc.), small bowel cancer (e.g., non-Hodgkin's lymphoma, gastrointestinal stromal tumor, etc.), breast cancer (e.g., invasive ductal carcinoma, non-invasive ductal carcinoma, inflammatory breast cancer, etc.), prostate cancer (e.g., hormone-dependent prostate cancer, hormone-independent prostate cancer, etc.), stomach cancer (e.g., papillary adenocarcinoma, mucinous adenocarcinoma, adenosquamous carcinoma, etc.), lung cancer (e.g., non-small cell lung cancer, small cell lung cancer, malignant mesothelioma, etc.), colon cancer (e.g., gastrointestinal stromal tumor, etc.), rectal cancer (e.g., gastrointestinal stromal tumor, etc.), esophageal cancer, duodenal cancer, tongue cancer, pharyngeal cancer (e.g., nasopharyngeal cancer, mesopharyngeal cancer, hypopharyngeal cancer, etc.), salivary gland cancer, brain tumors (e.g., astrocytic tumor of the pineal gland, ciliary astrocytoma, diffuse astrocytoma, anaplastic astrocytoma, etc.), schwannoma, liver cancer (e.g., primary liver cancer, extrahepatic bile duct cancer, etc.), kidney cancer (e.g., renal cell carcinoma, transitional epithelial carcinoma of the renal pelvis and ureter, etc.), cholangiocarcinoma, endometrial cancer, cervical cancer, ovarian cancer (e.g., epithelial ovarian cancer, extra-gonadal germ cell tumor, ovarian germ cell tumor, ovarian low-grade tumor, etc.), bladder cancer, urethral cancer, skin cancer (e.g., intraocular (eye) melanoma, Merkel cell carcinoma, etc.), hemangioma, malignant lymphoma, malignant melanoma, thyroid cancer (e.g., medullary thyroid cancer, etc.), parathyroid cancer, nasal cavity cancer, sinus cancer, bone tumors (e.g., osteosarcoma, Ewing tumor, uterine sarcoma, soft tissue sarcoma, etc.), angiofibroma, retinosarcoma, penile cancer, testicular tumor, pediatric solid tumor (e.g., Wilms tumor, pediatric renal tumor, etc.), Kaposi's sarcoma, Kaposi's sarcoma caused by AIDS, maxillary sinus tumor, fibrous histiocytoma, leiomyosarcoma, rhabdomyosarcoma, leukemia (e.g., acute myelogenous leukemia, acute lymphoblastic leukemia, etc.)

The pharmaceutical composition of the present invention may be administered in combination with other pharmaceuticals or therapeutic methods useful for the above-mentioned diseases. For example, malignant tumors may be combined with various types of chemotherapy, surgical treatment, and radiation therapy.

When the pharmaceutical composition of the present invention is administered to mammals (e.g., humans, mice, rats, guinea pigs, rabbits, dogs, horses, monkeys, pigs, etc.), especially humans, the dosage varies depending on the symptoms, patient age, sex, weight, susceptibility difference, administration method, administration interval, type of active ingredient, and type of formulation, and is not particularly limited. For example, 30 ug to 1000 mg, 100 ug to 500 mg, or 100 ug to 100 mg can be administered once or divided into several doses.

The examples shown below are merely examples and are intended only to explain the present invention in detail together with the above-described embodiments and are not intended to limit the present invention. Those skilled in the art can modify the present invention in various aspects without departing from the meaning of the present invention, and such modifications are also included in the scope of the present invention.

### Examples

The abbreviations used in this specification have the following meanings.
- K-Ras:: V-Ki-ras2 Kirsten rat sarcoma viral oncogene homolog
- N-Ras:: Neuroblastoma ras oncogene homolog
- H-Ras:: Harvey rat sarcoma viral oncogene homolog
- GDP:: Guanonsine diphosphate
- GTP:: Guanonsine triphosphate
- GEF:: Guanine nucleotide exchange factor
- GAP:: GTPase accelerating protein
- BSA:: Bovine serum albumin
- RP-HPLC:: Reverse-phase high performance liquid chromatography
- HRP:: Horseradish peroxidase
- SA:: Streptavidin
- DTT:: Dithiothreitol
- PBS:: Phosphate buffered saline
ELISA: Enzyme-linked immunosorbent assay
ATP: Adenosine triphosphate
Ac: Acetyl
Arg: L-Arginine
Cys: L-Cysteine
Gly: Glycine
Ala: L-Alanine
βAla: beta-Alanine (beta-homo-Glycine)
γAba: gamma-aminobutyric acid
Aze: L-Azetidine carboxylic acid
Pro: L-Proline
t4fP: Trans-4-fluoro-L-Proline
Pip: L-Pipecolic acid
Val: L-Valine
Leu: L-Leucine
Ile: L-Isoleucine
γmL: gamma-methyl-L-Leucine (3-tert-butyl-L-Alanine)
Nle: L-Norleucine
dmNl: 5,5-dimethyl-L-Norleucine
Ahep: (S)-2-Aminoheptanonic acid
Aoc: (S)-2-Aminooctanonic acid
Anon: (S)-2-Aminononanonic acid
Adec: (S)-2-Aminodecanoic acid
Cprg: L-Cyclopropylglycine
Cbug: L-Cyclobutylglycine
Cpeng: L-Cyclopentylglycine
Chg: L-Cyclohexylglycine
Cha: L-Cyclohexylalanine
Phg: L-Phenylglycine
Phe: L-Phenylalanine
3cPg: 3-chloro-L-Phenylglycine
2cPg: 2-chloro-L-Phenylglycine
4fPg: 4-fluoro-L-Phenylglycine
Tyr: L-Tyrosine
3hF: 3-hydroxy-L-Phenylalanine
Trp: L-Tryptophan
5hW: 5-hydroxy-L-Tryptophan
6cW: 6-chloro-L-Tryptophan
1NaphA: L-1-Naphthylalanine
- 2NaphA:: L-2-Naphthylalanine
- Dap:: (S)-2,3-Diaminopropanonic acid
- Lys:: L-Lysine
- S(Ome):: O-methyl-L-Serine
- hmS(Ome):: O-methyl-L-homoserine
- Thr:: L-Threonine
- Asp:: L-Aspartic acid
- 4fF:: 4-fluoro-L-Phenylalanine
- 4mF:: 4-methyl-L-Phenylalanine
- 4cF:: 4-chloro-L-Phenylalanine
- 4tfmF:: 4 -trifluoromethyl-L-Phenylalanine
- 4bF:: 4-bromo-L-Phenylalanine
- 4iF:: 4-iodo-L-Phenylalanine
- 2PyrdA:: 3-(2-Pyridyl)-L-Alanine
- Tic:: (S)-1,2,3,4-tetrahydroisoquinoline-3-carboxylic acid
- DIE:: 1,2-Diiodoethane
- DIP:: 1,3-Diiodopropane
- DIB:: 1,4-Diiodobutane
- ^{m}X:: N-methylated amino acid X
- ^{D}X:: D-amino acid X
- c[XY],c(XY):: Cyclization between amino acid X to amino acid Y

### (Peptide synthesis)

The chemical synthesis of all the peptides used in this example was outsourced to Scrum Inc. (Tokyo, Japan) and was carried out using a standard solid phase synthesis method using 9-fluorenylmethoxycarbonyl group (Fmoc group) as a protecting group for the α-amino group on an automated SyroII synthesis machine (Biotage). A side-chain protected amino acid-resin located at the C-terminus was placed in the synthesis column and the apparatus was set up. To the next amino acid protected by the Fmoc group, 1-[bis(dimethylamino)methylene]-1H-1,2,3-triazolo[4,5-b]pyridini um 3 -oxide hexafluorophosphate (HATU) /diisopropylethylamine (DIEA) was added to activate and placed in a column. After completion of the coupling reaction, the column was washed and the Fmoc group was deprotected using 20% piperidine. The peptide chain was extended by repeating this process, and after deprotection of the Fmoc group of the final amino acid, the peptide resin was removed from the apparatus. The linear side chain protected peptide was cut out from the resin by adding 30% hexafluoro-2-propanol (HFIP)/dichloromethane (DCM) to the peptide resin, and the side chain protected peptide was recovered by ether precipitation. The side-chain protected peptides were purified by RP-HPLC using a SunFire C18 column (10x150 mm) (Waters) and lyophilized.

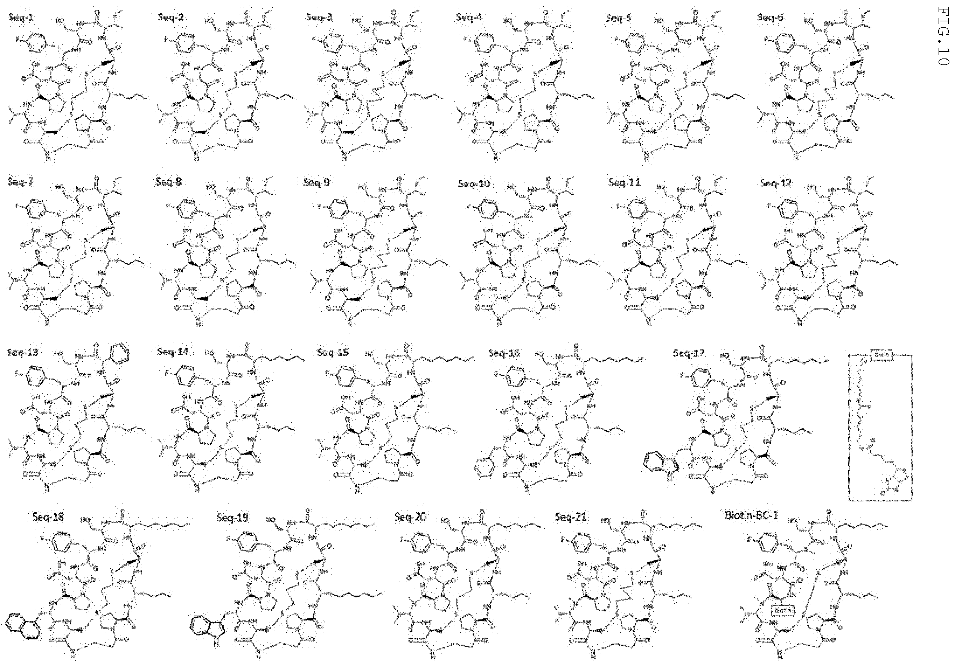

The cyclization of peptides was based on the methods described in Non-Patent Literature 3, Non-Patent Literature 6 and Non-Patent Literature 7. As an example, the cyclization of Seq-21 (LO3) is shown below. The linear side-chain protected peptide was dissolved in DCM, and 1-hydroxybenzotriazole (HOAt) and ethyl (dimethylaminopropyl) carbodiimide (EDC) hydrochloride dissolved in dimethylformamide (DMF) were added to activate the C-terminal carboxy group. After 1 hour of reaction on ice, the peptide was further reacted overnight at room temperature to cyclize the side-chain protected peptide by an amide bond between the N-terminal amino group and the C-terminal carboxy group. The monocyclic peptides were obtained by washing three times with distilled water, distilling off DCM, drying, deprotection of side chain protecting groups, and ether precipitation. The monocyclic peptides were purified by RP-HPLC using a SunFire C18 column (10x150 mm) (Waters) and lyophilized. The monocyclic peptides were dissolved in dimethyl sulfoxide (DMSO) and added to a mixture of 0.1 M NaHCO₃ and acetonitrile containing 1,4-diiodobutane (DIB) dissolved in DMF and 10 mM tris (2-carboxyethyl)phosphine (TCEP). The peptide was cyclized by the thioether bond between the thiol group and the alkyl halide linker by adding it to the mixture and reacting at 80°C. The bicyclic peptide LO3 was purified by RP-HPLC using a SunFire C18 column (10x150 mm) (Waters) and then lyophilized. The molecular weight of the final peptide was measured using microflex (Bruker) to identify the target product. The theoretical molecular weight, the measured molecular weight, purity, cyclization type, and amino acid sequence of the peptides synthesized in this example are shown in Table 2. Among them, the structural formula of Biotin-BC-1, sequences 1-21 (Seq-1 to Seq-21) is shown in Fig. 10. In Table 2, amino acids without the D-amino acids notation indicate the L-amino acids.

### (Construction of a competitive binding test by ELISA)

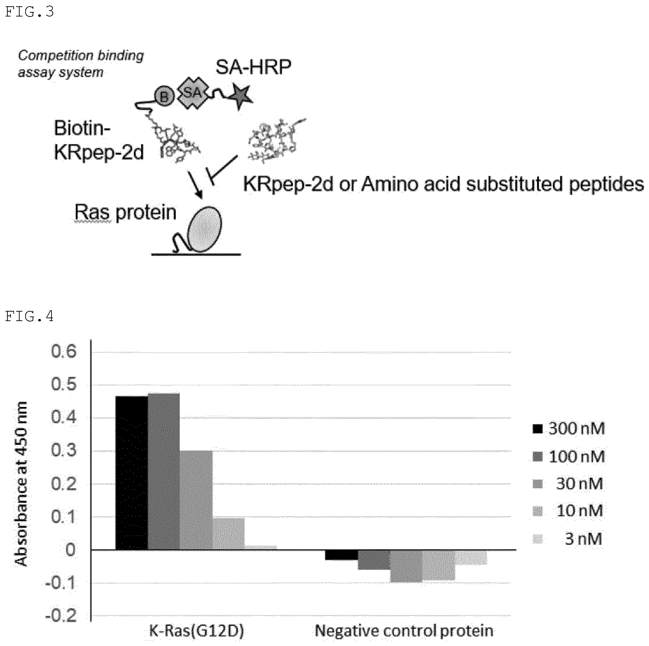

In order to compare the binding activity of the amino acid-substituted peptide to Ras with that of KRpep-2d, we constructed a competitive binding test using the ELISA shown in Fig. 3. The ELISA method is briefly described below. First, the recombinant protein of Ras was added to 96-well Maxi-Soap plates (Catalog No. 439454, Nunc) at 12.5 ng/50 µL/well using 10 mM MgCl₂/10 µM GDP/PBS and coated overnight at 4°C. Then, 0.1% BSA/10 mM MgCl₂/10 µM GDP/PBS was added at an additional 300 µL/well and blocked for 30 minutes at room temperature. After washing the plate with 0.1% Tween20/PBS, 0.025% BSA/0.05% Tween20/10mM MgCl₂/10 µM GDP/PBS was used to prepare a mixture of Biotin-KRpep-2d (100 nM) and any concentration of amino acid substituted peptide or KRpep-2d as a comparison and added at 50 µL/well. After the reaction for 30 min at room temperature, the plate was washed with 0.1% Tween 20/PBS. Biotin-KRpep-2d bound to Ras coated on the plate was detected by SA-HRP (Catalog No. ab7403, Abcam). For the quantification of HRP, TMB-ELISA Substrate Solution (Catalog No. 34028, manufactured by Thermo Fisher Scientific Co., Ltd.) was used to measure the absorbance at 450 nm. The binding of Biotin-KRpep-2d to Ras competes with the binding of amino acid-substituted peptide or KRpep-2d to Ras coexisting in solution, and thus is inhibited depending on the concentration of the amino acid-substituted peptide or KRpep-2d. That is, the binding activity of an amino acid-substituted peptide or KRpep-2d to Ras is detected as a competitive inhibitory activity to the binding of Biotin-KRpep-2d. Under the assumption that the binding value of Biotin-KRpep-2d to wells without Ras coating is taken as 100% inhibitory activity, and the binding value of Biotin-KRpep-2d to wells without amino acid substituted peptide or KRpep-2d is taken as 0% inhibitory activity, the 50% inhibitory concentration (IC₅₀) values of amino acid substituted peptide and KRpep-2d were calculated. Then, the relative values of the inhibitory activity of the amino acid substituted peptides were calculated with the IC₅₀ value of KRpep-2d as 1.

FIG. 4 shows the peptide concentration-dependent binding of Biotin-KRpep-2d to K-Ras(G12D) (Catalog No. 12259-H07E1-200, manufactured by Sino Biological) used in this competitive test. The specific binding activity was estimated by subtracting the absorbance obtained when Biotin-KRpep-2d was added to wells blocked only with BSA from the absorbance obtained when Biotin-KRpep-2d was added to wells coated with K-Ras(G12D). As a result, Biotin-KRpep-2d showed a concentration-dependent binding of the peptide to K-Ras (G12D) . On the other hand, Biotin-KRpep-2d did not show binding activity to negative control proteins.

### (Evaluation of binding activity of amino acid-substituted peptide to K-Ras(G12D))

The results of the competitive binding test against K-Ras(G12D) with N=4 are shown in Table 1. Most of the peptide sequences with one or two amino acid substitutions for KRpep-2d showed the same or stronger binding activity than KRpep-2d, indicating that these amino acid substitutions are effective in improving the binding activity of the peptides against K-Ras(G12D).

2d-5/15-Dap/Asp, in which Cys at position 5 and Cys at position 15 were replaced with Dap and Asp, respectively, and cyclized by amide bond between them, showed K-Ras (G12D) binding activity, even though it was slightly weakened to 0.6-fold of KRpep-2d.

**[Table 1-1]**

| Name | Substituted position | Introduced amino acid | Binding intensity (fold ± S.E.) |
|---|---|---|---|
| 2d-6-t4fP | Pro⁶ | t4fP | 1.8 ± 0.1 |
| 2d-6-Pip | Pro⁶ | Pip | 1.4 ± 0.0 |
| 2d-6-^{m}Nle | Pro⁶ | ^{m}Nle | 2.7 ± 0.0 |
| 2d-6-^{m}Gly | Pro⁶ | ^{m}Gly | 0.7 ± 0.1 |
| 2d-7-Nle | Leu⁷ | Nle | 1.5 ± 0.1 |
| 2d-7-γmL | Leu⁷ | γmL | 1.0 ± 0.1 |
| 2d-7-dmNI | Leu⁷ | dmNI | 1.8 ± 0.2 |
| 2d-7-Ahep | Leu⁷ | Ahep | 2.2 ± 0.2 |
| 2d-7-Aoc | Leu⁷ | Aoc | 4.0 ± 0.1 |
| 2d-7-Anon | Leu⁷ | Anon | 5.9 ± 0.2 |
| 2d-7-Adec | Leu⁷ | Adec | 8.4 ± 0.2 |
| 2d-7-Cprg | Leu⁷ | Cprg | 0.6 ± 0.0 |
| 2d-7-Cbug | Leu⁷ | Cbug | 1.2 ± 0.1 |
| 2d-7-Cpeng | Leu⁷ | Cpeng | 0.6 ± 0.0 |
| 2d-7-Chg | Leu⁷ | Chg | 1.7 ± 0.1 |
| 2d-7-Phg | Leu⁷ | Phg | 1.7 ± 0.0 |
| 2d-7-3cPg | Leu⁷ | 3cPg | 2.6 ± 0.3 |
| 2d-7-2cPg | Leu⁷ | 2cPg | 1.9 ± 0.1 |
| 2d-7-4fPg | Leu⁷ | 4fPg | 2.2 ± 0.2 |
| 2d-8-3hF | Tyr⁸ | 3hF | 2.1 ± 0.0 |
| 2d-8-Trp | Tyr⁸ | Trp | 2.6 ± 0.1 |
| 2d-8-1NaphA | Tyr⁸ | 1NaphA | 6.5 ± 0.9 |
| 2d-8-2NaphA | Tyr⁸ | 2NaphA | 5.9 ± 0.8 |
| 2d-8-6cW | Tyr⁸ | 6cW | 9.8 ± 0.1 |
| 2d-8-5hW | Tyr⁸ | 5hW | 2.5 ± 0.2 |
| 2d-9-^{m}Nle | Ile⁹ | ^{m}Nle | 1.3 ± 0.1 |
| 2d-9-Nle | Ile⁹ | Nle | 1.2 ± 0.1 |
| 2d-9-Ahep | Ile⁹ | Ahep | 2.6 ± 0.1 |
| 2d-9-Aoc | Ile⁹ | Aoc | 5.4 ± 0.3 |
| 2d-9-Anon | Ile⁹ | Anon | 8.3 ± 0.2 |
| 2d-9-Adec | Ile⁹ | Adec | 9.6 ± 0.4 |
| 2d-9-Cprg | Ile⁹ | Cprg | 0.7 ± 0.0 |
| 2d-9-Cbug | Ile⁹ | Cbug | 1.4 ± 0.0 |
| 2d-9-Cpeng | Ile⁹ | Cpeng | 1.7 ± 0.0 |
| 2d-9-Chg | Ile⁹ | Chg | 2.7 ± 0.1 |
| 2d-9-Phg | Ile⁹ | Phg | 2.4 ± 0.1 |

**[Table 1-2]**

| Name | Substituted position | Introduced amino acid | Binding intensity (fold ± S.E.) |
|---|---|---|---|
| 2d-10-^{m}Ser | Ser¹⁰ | ^{m}Ser | 0.7 ± 0.0 |
| 2d-10-S(Ome) | Ser¹⁰ | S(Ome) | 0.7 ± 0.0 |
| 2d-10-hmS(Ome) | Ser¹⁰ | hmS(Ome) | 0.7 ± 0.0 |
| 2d-10-Dap | Ser¹⁰ | Dap | 4.8 ± 0.0 |
| 2d-10-Thr | Ser¹⁰ | Thr | 0.9±0.1 |
| 2d-10-Val | Ser¹⁰ | Val | 1.2 ± 0.1 |
| 2d-10-Leu | Ser¹⁰ | Leu | 2.5 ± 0.1 |
| 2d-11-^{m}Tyr | Tyr¹¹ | ^{m}Tyr | 1.1 ± 0.1 |
| 2d-11-2PyrdA | Tyr¹¹ | 2PyrdA | 0.4 ± 0.1 |
| 2d-11-4fF | Tyr¹¹ | 4fF | 1.8 ± 0.0 |
| 2d-11-4mF | Tyr¹¹ | 4mF | 2.8 ± 0.2 |
| 2d-11-4cF | Tyr¹¹ | 4cF | 4.1 ± 0.2 |
| 2d-11-4tfmF | Tyr¹¹ | 4tfmF | 7.6 ± 0.2 |
| 2d-11-4bF | Tyr¹¹ | 4bF | 5.3 ± 0.6 |
| 2d-11-4iF | Tyr¹¹ | 4iF | 6.7 ± 0.8 |
| 2d-11-Trp | Tyr¹¹ | Trp | 2.6 ± 0.1 |
| 2d-11-^{m}Trp | Tyr¹¹ | ^{m}Trp | 2.7 ± 0.0 |
| 2d-11-2NaphA | Tyr¹¹ | 2NaphA | 8.1 ± 0.2 |
| 2d-11-6cW | Tyr¹¹ | 6cW | 7.6 ± 0.6 |
| 2d-11-Cha | Tyr¹¹ | Cha | 4.2 ± 0.0 |
| 2d-13-Tic | Pro¹³ | Tic | 4.4 ± 0.2 |
| 2d-13-^{m}Trp | Pro¹³ | ^{m}Trp | 4.3 ± 0.1 |
| 2d-13-^{m}Gly | Pro¹³ | ^{m}Gly | 0.9 ± 0.1 |
| 2d-14-^{m}Val | Val¹⁴ | ^{m}Val | 1.4 ± 0.1 |
| 2d-14-2PyrdA | Val¹⁴ | 2PyrdA | 1.6 ± 0.3 |
| 2d-14-Trp | Val¹⁴ | Trp | 3.4 ± 0.5 |
| 2d-14-^{m}Trp | Val¹⁴ | ^{m}Trp | 2.3 ± 0.0 |
| 2d-14-1NaphA | Val¹⁴ | 1NaphA | 7.0 ± 0.3 |
| 2d-14-Nle | Val¹⁴ | Nle | 1.3 ± 0.1 |
| 2d-13/14-Tic/Trp | Pro¹³/Val¹⁴ | Tic/Trp | 9.4 ± 0.1 |
| 2d-13/14-Tic/1NaphA | Pro¹³/Val¹⁴ | Tic/1NaphA | 9.6 ± 0.1 |
| 2d-5/15-Dap/Asp | CYS⁵/CyS¹⁵ | Dap⁵/Asp¹⁵ | 0.6 ± 0.0 |

### (Evaluation of binding activity of the peptide of the present invention to Ras)

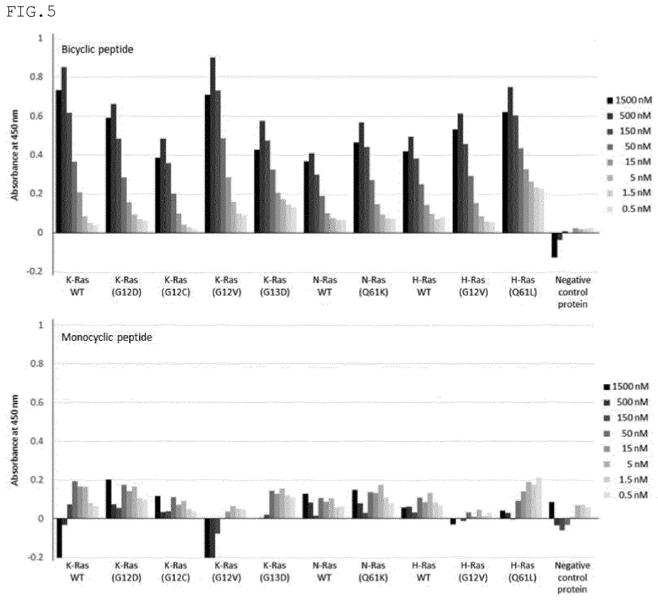

Biotin-BC-1 is a biotin-modified form of a representative example of an inventive peptide in which an unnatural amino acid is introduced into KRpep-2d, and the contiguous arginine sequence is further removed and bicycled by covalent bonds between positions 1 and 11 and positions 4 and 11. The peptide concentration-dependent bindings of Biotin-BC-1 to wild-type K-Ras, K-Ras (G12D), K-Ras (G12V), K-Ras(G12C), K-Ras(G13D), wild-type N-Ras, N-Ras(Q61K), wild-type H-Ras, H-Ras(G12V), and H-Ras(Q61L) are shown in FIG. 5.

As shown in Fig.5, the biotin-modified peptide, which is bicycled by covalent bonds between positions 1 and 11 and positions 4 and 11, showed concentration-dependent binding of the peptide to all the Ras proteins. On the other hand, no binding activity of the peptide was observed to the negative control protein. The binding activity of the biotin-modified peptide which becomes monocyclic with only the covalent bond between positions 1 and 11 by cleaving the covalent bond between positions 4 and 11 under the reducing condition, was greatly weakened for all the Ras proteins.

### (Evaluation of resistance of the inventive peptides to protease degradation)

To confirm that the inventive peptides are more resistant to protease degradation than KRpep-2d, KRpep-2d or the peptides synthesized in this study (Seq-17, Seq-20 (LO2) and Seq-21 (LO3)) were mixed in rat plasma, and the peptides remaining after a certain time of incubation were evaluated by the presence or absence of RP-HPLC peaks. For 1 µL of each 10 mM peptide, 20 µL of rat plasma was added and incubated at 37°C for an arbitrary time (0 or 24 hours) . Then, 200 µL of 80% methanol was added to KRpep-2d, and 200 µL of acetonitrile was added to the synthetic peptides (Seq-17, Seq-20, and Seq-21), and after mixing well, the mixture was placed on ice for 10 min. To remove plasma proteins, the mixture was centrifuged at a speed of 15,000 rpm under 4°C for 10 min. The supernatant was collected and the remaining amount of peptide was detected by RP-HPLC (solution A: 0.1% TFA/water, solution B: 0.1% TFA/acetonitrile, 20 min gradient of 80% solution A→10% solution A at 1 mL/min) using a SunFire C18 column (5 *µ*m, 4.6X150 mm).

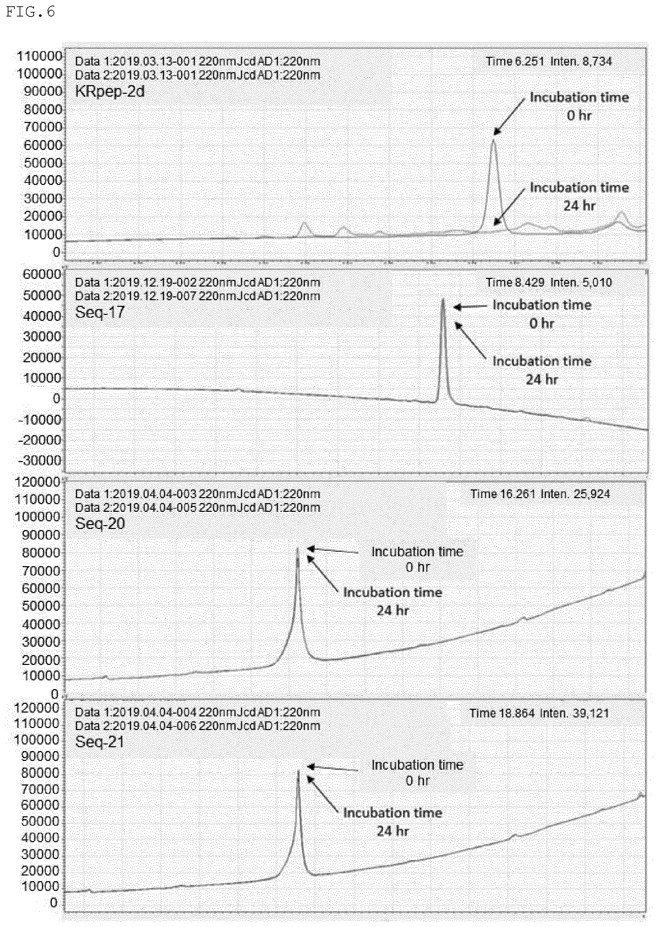

The results are shown in FIG.6. The KRpep-2d-derived peak disappeared after 24 hours of incubation, suggesting that some part of the sequence underwent protease degradation. On the other hand, the peaks derived from synthetic peptides (Seq-17, Seq-20, and Seq-21) remained almost the same even after 24 hours of incubation compared to 0 hours, indicating that they were resistant to protease degradation.

### (Evaluation of growth inhibitory activity of the peptides against mutant K-Ras expressing cells)

To confirm that the peptides of the present invention inhibit Ras signaling in cells and consequently inhibit cell proliferation, a cell proliferation test was performed with N=4. The following is a brief description of the cell proliferation test, which may be performed according to the Non-Patent Literature 3. A427 cells expressing K-Ras(G12D) (Catalog No. HTB-53, ATCC) or PANC-1 cells expressing K-Ras (G12D) (Catalog No. CRL-1469, ATCC) were spread into 96-well plates at 1×10³ cells/well and cultured overnight at 37°C in a CO₂ incubator. Peptides diluted in serum medium (final concentration 30 µM) were added to the wells, and after culturing for 24 hours, all the medium was removed, and then freshly diluted peptides in serum medium (final concentration 30 µM) were added to the wells and incubated for 24 hours. The number of cells in the wells was quantified by CellTiter-Glo (Catalog No. G7570, Promega) after the removal of medium and addition of peptide for a total of three times. The number of cells in the wells before the addition of the peptide was used as the growth inhibition rate (100%), and the number of cells in the wells without the peptide after the entire process was used as the growth inhibition rate (0%), and the growth inhibition activity of any concentration of the peptide was calculated.

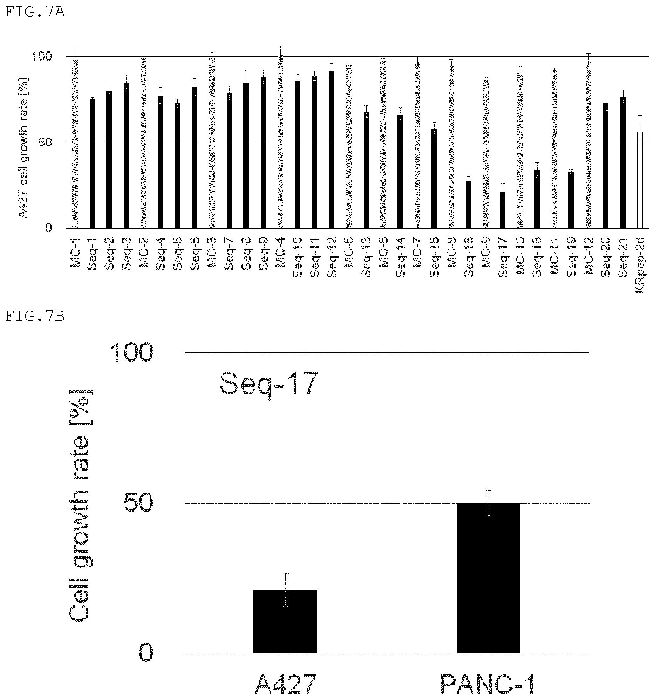

The results are shown in Figs.7 (a) and 7(b). As shown in Fig.7(A), the peptides synthesized in the example (sequences 1-21) inhibited the proliferation of A427 cells. On the other hand, the growth inhibitory activity of the monocyclic peptide, MC1-12, against A427 cells was greatly attenuated compared to those of the bicyclic peptides. As shown in Fig.7 (B), the peptides of Seq-17 inhibited the proliferation of PANC-1 cells expressing K-Ras (G12D) as well as A427 cells.

### (Evaluation of anticancer activity of peptides of the present invention in subcutaneous carcinoma-bearing mice)

We evaluated whether the peptides of the present invention exhibit anticancer activity *in vivo.* PANC-1 cells expressing K-Ras(G12D) (Catalog No. CRL-1469, ATCC) were transplanted at 1×10⁷ cells under the left inguinal skin of Crl:SHO-Prkdc^{scid}Hr^{hr} mice (female, 7 weeks old) (Charles River, Japan) . When the tumor volume reached approximately 50 mm³, the mice were submitted to the study. Tumor volume was determined by measuring the long diameter (mm) and the short diameter (mm) orthogonal to the long diameter from the skin surface with calipers and using the formula: (long diameter) x (short diameter) ² × 0.5, with the unit being mm³. A representative example of the inventive peptide (Seq-17) was dissolved in DMSO, diluted 10 times with saline, and administered at 40 mg/kg through the tail vein once every two days, and the body weight and tumor volume of mice were measured over time (peptide administration group: N=10, vehicle group: N=4). The vehicle group was the group in which cancer cells were implanted and saline (10% DMSO) was administered.

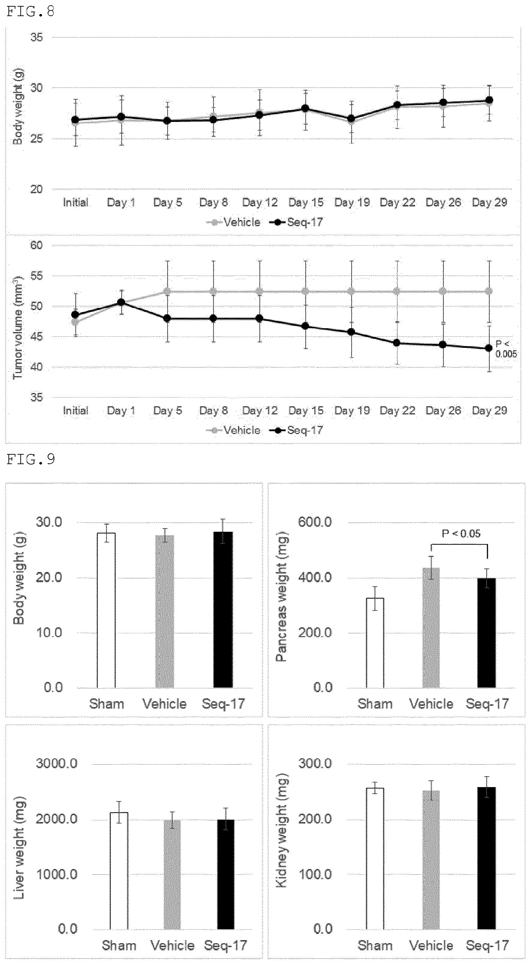

The results are shown in Fig.8. In mice treated with a representative example of the inventive peptide (Seq-17), the increase in tumor volume was significantly suppressed, and the p-value calculated by Student's t-test was less than 0.005. On the other hand, there was no decrease in the body weight of the mice.

### (Evaluation of anti-cancer activity of the peptide of the present invention in orthotopic transplant model mice)

We evaluated whether the peptides of the present invention exhibit anticancer activity *in vivo.* PANC-1 cells expressing K-Ras (G12D) (Catalog No. CRL-1469, ATCC) were transplanted at 2.5×10⁶ cells under the tail capsule of the pancreas of BALB/cAJcl-nu/nu mice (male, 8 weeks old) (Nihon Clare Co., Ltd.), and were used for the test one week later. The peptide (Seq-17) synthesized in this study was dissolved in DMSO, diluted 10-fold with saline, and administered at 40 mg/kg through the tail vein once every two days, and the body weight of the mice was measured over time (Sham group, peptide administration group, and vehicle group: N=7 for each) . The Sham group was the group that received saline (10% DMSO) without cancer cell transplantation, and the Vehicle group was the group that received saline (10% DMSO) with cancer cell transplantation. The weight of the mice and the weight of their organs (pancreas, liver, and kidney) were measured four weeks after the start of administration.

The results are shown in Fig.9. In the mice treated with the synthetic peptide (Seq-17), the increase in the weight of pancreas derived from the proliferation of PANC-1 cells was significantly suppressed, and the p-value calculated by Student's t-test was less than 0.05. On the other hand, there was no significant difference in the weight of liver and kidney. In addition, there was no decrease in the body weight of the mice.

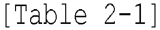

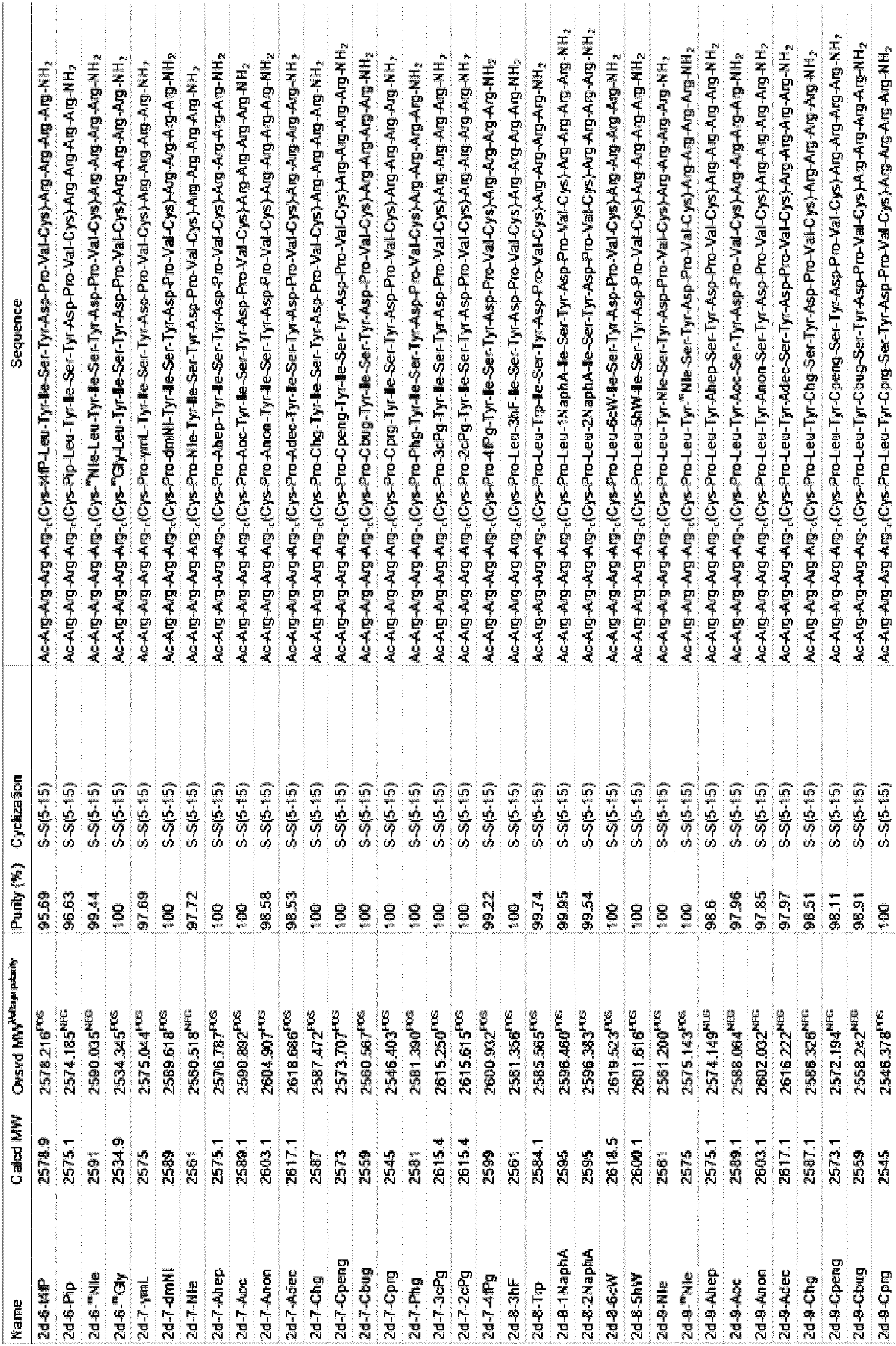

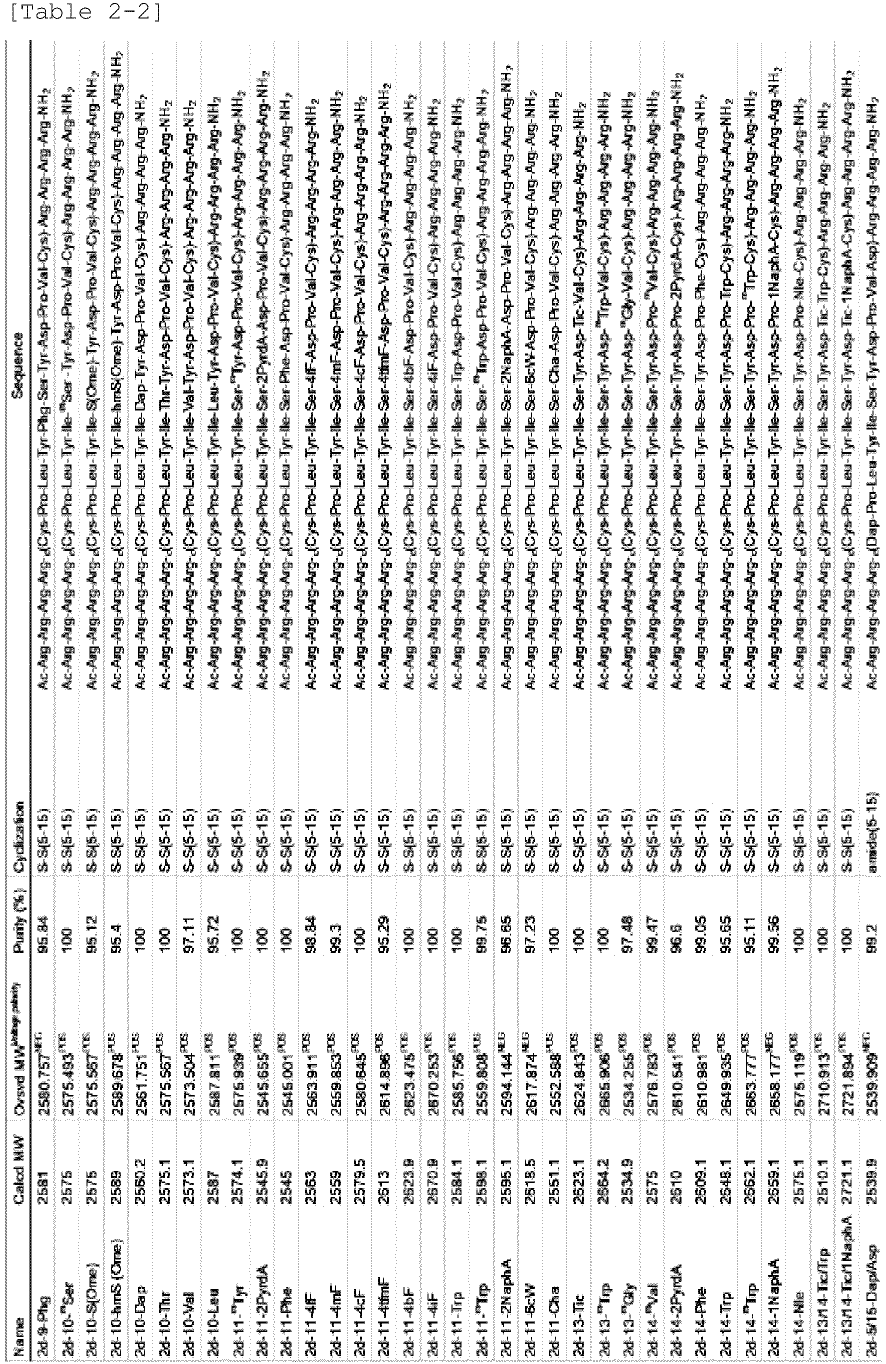

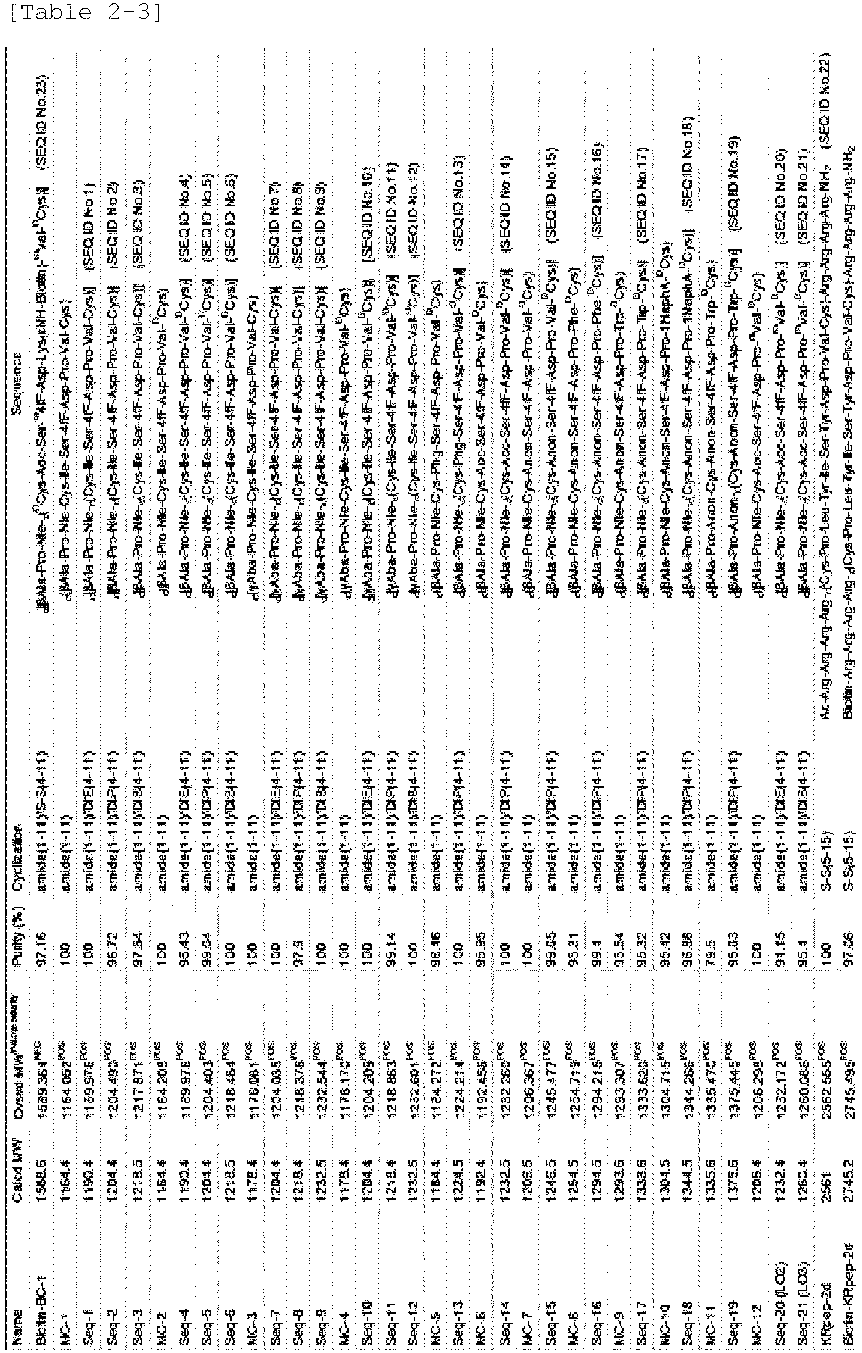

### Industrial applicability

The peptides of the present invention are resistant to protease degradation, and the binding of the peptides to Ras inhibits the off/on cycle of the molecular switch function of Ras, thereby suppressing the growth of cancer cells that use Ras as a growth driver. Therefore, the peptides may be useful in the prevention and treatment of diseases involving cancer cells expressing Ras, such as colon cancer, pancreatic cancer, and lung cancer.

The peptides can be used not only as anticancer agents themselves, but also as medicinal molecules combined with delivery molecules to cancer tissues such as antibodies, lectins, glycans, liposomes, and nanoparticles. It can also be used as a functional molecule that selectively degrades intracellular Ras by combining it with molecules that ubiquitinate proteins and induce them to degrade.

Furthermore, the peptides of the present invention may be able to inhibit the growth of cancer cells more potently when used in combination with drugs and therapies with other mechanisms of action, such as immune checkpoint inhibitors and neutron supplementation therapy.

## Claims

1. A cyclic peptide comprising an amino acid sequence represented by the following formula (1):
c[X¹-X²-X³-c(X⁴-X⁵-X⁶-X⁷-X⁸-X⁹-X¹⁰-X¹)] (1)
wherein
X¹, X⁴ and X¹¹, each independently denotes an amino acid residue having an amino group, a carboxyl group, a thiol group, an allyl group, an alkynyl group, an azido group or a halogen atom, or a derivative thereof;
X³, X⁵ and X⁷, each independently denotes an amino acid residue having a hydrocarbon group optionally having a substituent, or an aromatic heterocyclic group optionally having a substituent, or a derivative thereof;
X⁸ denotes an aspartic acid residue or a derivative thereof;
X², X⁶, X⁹ and X¹⁰, each denotes any amino acid residue;
X¹ and X¹¹, and X⁴ and X¹¹, independently of each other, covalently bind directly or via a linker to form two cyclic structures in the peptide molecule of formula (1);
the covalent bond between X¹ and X¹¹ is any linkage between main-chains, a main-chain and a side-chain, a side-chain and a main-chain or side-chains thereof; and
the covalent bond between X⁴ and X¹¹ is any linkage between a side-chain and a main-chain or side-chains thereof;
or a pharmaceutically acceptable salt thereof.

2. The cyclic peptide according to claim 1 or a pharmaceutically acceptable salt thereof, wherein
a linkage between Cα carbon atoms of X¹ and X¹¹ comprises a structure represented by the following formula (2):
C_{α}¹- (CH₂)_{A}-X- (CH₂)_{B}-Cα¹¹ (2)
wherein
A and B each independently denotes an integer from 0 to 2;
a sum of A and B is an integer from 1 to 4; and
X is S-S, CH₂-CH₂, S-CH₂, CH₂-S, O-CH₂, CH₂-O, CH=CH, NH-C(=O), N (CH₃)-C (=O) , NH-C(=S), N(CH₃)-C(=S), O-C(=O), C(=O)-NH, C (=O)-N (CH₃), C (=S) -NH, C (=S) -N (CH₃), C (=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C (=CH₂)-S, S- (CH₂)₂-S, S- (CH₂)₃-S, S- (CH₂)₄-S, S-CH₂-CH=CH-CH₂-S, S-CH₂-C(=O)-NH, NH-C(=O)-CH₂-S, S-CH₂-C₆H₄-CH₂-S, S-CH₂-C(=O) -CH₂-S or S-CH₂-C(=CH₂)-CH₂-S.

3. The cyclic peptide according to claim 1 or 2, or a pharmaceutically acceptable salt thereof, wherein
a linkage between Cα carbon atoms of X⁴ and X¹¹ comprises a structure represented by the following formula (3):
Cα⁴- (CH₂)_{A}-Y- (CH₂)_{B}-Cα¹¹ (3)
wherein
A and B each independently denotes an integer from 0 to 4;
a sum of A and B is an integer from 2 to 4; and
Y is S-S, CH₂-CH₂, S-CH₂, CH₂-S, O-CH₂, CH₂-O, CH=CH, NH-C(=O), N(CH₃)-C(=O) , NH-C(=S), N(CH₃)-C(=S), O-C(=O), C(=O)-NH, C(=O)-N(CH₃), C(=S) -NH, C(=S)-N(CH₃), C(=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C (=CH₂)-S, S- (CH₂)₂-S, S- (CH₂)₃-S, S- (CH₂)₄-S, S-CH₂-CH=CH-CH₂-S, S-CH₂-C(=O) -NH, NH-C(=O)-CH₂-S, S-CH₂-C₆H₄-CH₂-S, S-CH₂-C(=O) -CH₂-S S-CH₂-C(=CH₂)-CH₂-S, triazole or dithio-tetrafluorobenzene.

4. The cyclic peptide according to any one of claims 1 to 3, or a pharmaceutically acceptable salt thereof, wherein
X³, and X⁵, each independently denotes an amino acid residue represented by the following formula (4): 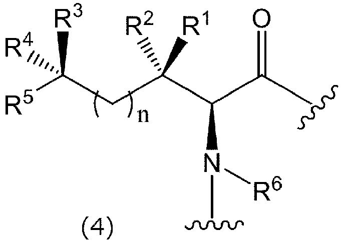 wherein a wavy line denotes the point of attachment to the carbonyl group or the nitrogen atom that form the main chain amide bond; R¹, R² and R⁶, each independently denotes a hydrogen atom or a methyl group; R³, R⁴ and R⁵, each independently denotes a hydrogen atom, a methyl group or a halogen atom (F, Cl, Br, I); and n denotes an integer of 0 to 10; or
the following formula (5): 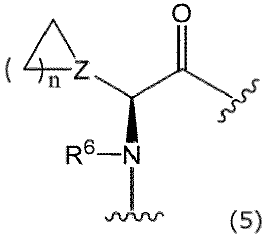 wherein a wavy line denotes the point of attachment to the carbonyl group or the nitrogen atom that form the main chain amide bond; Z denotes C or N; R⁶ denotes a hydrogen atom or a methyl group; and n denotes an integer of 1 to 6; or
the following formula (6): 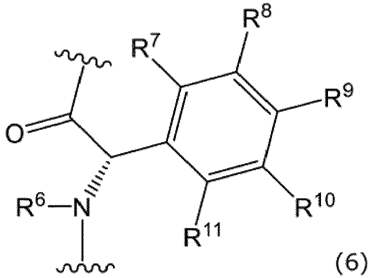 wherein a wavy line denotes the point of attachment to the carbonyl group or the nitrogen atom that form the main chain amide bond; R⁷, R⁸, R⁹, R¹⁰ and R¹¹, each independently denotes a hydrogen atom or a halogen atom (F, Cl, Br, I); and R⁶ denotes a hydrogen atom or a methyl group; or
the following formula (7): 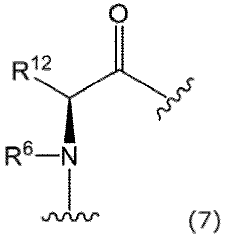 wherein a wavy line denotes the point of attachment to the carbonyl group or the nitrogen atom that form the main chain amide bond; R¹² denotes a 2-thienyl group, 3-thienyl group, 2-pyridyl group, 3-pyridyl group, 4-pyridyl group, 2-thiazolyl group, 4-thiazolyl group, 2-oxazolyl group, 4-oxazolyl group, 1-pyrazolyl group, 1-imidazolyl group, 1,2,3,-triazol-1-yl group, or 1,2,4-triazol-1-yl group; R⁶ denotes a hydrogen atom or a methyl group;
or a derivative thereof.

5. The cyclic peptide according to any one of claims 1 to 4, or a pharmaceutically acceptable salt thereof, wherein
X⁷ denotes an amino acid residue represented by the following formula (8): 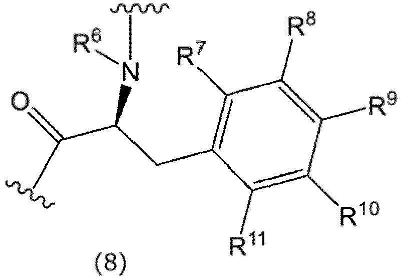 wherein a wavy line denotes the point of attachment to the carbonyl group or the nitrogen atom that form the main chain amide bond; R⁷, R⁸, R⁹, R¹⁰ and R¹¹, each independently denotes a hydrogen atom or a halogen atom (F, Cl, Br, I); and R⁶ denotes a hydrogen atom or a methyl group; or
the following formula (9): 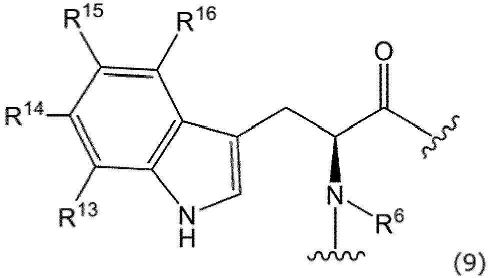 wherein a wavy line denotes the point of attachment to the carbonyl group or the nitrogen atom that form the main chain amide bond; R¹³, R¹⁴, R¹⁵ and R¹⁶, each independently denotes a hydrogen atom, a hydroxy group, a methoxy group, a methyl group, a tert-butyl group, a halogenated methyl group or a halogen atom (F, Cl, Br, I); and R⁶ denotes a hydrogen atom or a methyl group;
the following formula (10): or 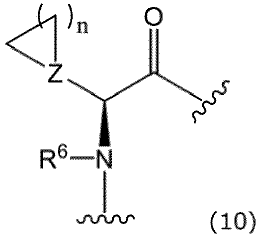 wherein a wavy line denotes the point of attachment to the carbonyl group or the nitrogen atom that form the main chain amide bond; Z denotes C or N; R⁶ denotes a hydrogen atom or a methyl group; and n denotes an integer of 1 to 6;
the following formula (11): 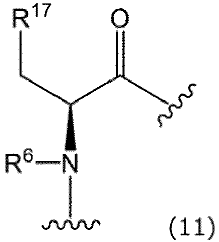 wherein a wavy line denotes the point of attachment to the carbonyl group or the nitrogen atom that form the main chain amide bond; R¹⁷ denotes a 2-thienyl group, 3-thienyl group, 2-pyridyl group, 3-pyridyl group, 4-pyridyl group, 2-thiazolyl group, 4-thiazolyl group, 2-oxazolyl group, 4-oxazolyl group, 1-pyrazolyl group, 1-imidazolyl group, 1,2,3,-triazol-1-yl group, or 1,2,4-triazol-1-yl group, 1H-benzoimidazol-1-yl group, 1H-benzoimidazol-2-yl group, 1,3-benzothiazol -2-yl group, 1,3-benzooxazol-2-yl group, 2-quinolyl group, 8-quinolyl group, 1-naphtyl group or 2-naphtyl group; R⁶ denotes a hydrogen atom or a methyl group;
or a derivative thereof.

6. The cyclic peptide according to any one of claims 1 to 5, or a pharmaceutically acceptable salt thereof, wherein
X⁶ denotes an amino acid residue represented by the following formula (12): 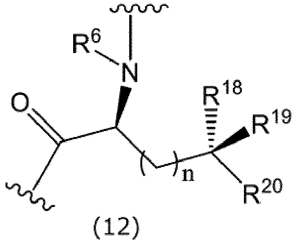 wherein a wavy line denotes the point of attachment to the carbonyl group or the nitrogen atom that form the main chain amide bond; R¹⁸, R¹⁹ and R²⁰, each independently denotes a hydrogen atom a hydroxy group, a methoxy group, an amino group, a monomethylated amino group, a dimethylated amino group, a trimethylated amino group, a methyl group, an ethyl group, an isopropyl group, a tert-butyl group, a halogenated methyl group or a halogen atom (F, Cl, Br, I); R⁶ denotes a hydrogen atom or a methyl group; and n denotes an integer of 0 or 1;
or a derivative thereof.

7. The cyclic peptide according to any one of claims 1 to 6, or a pharmaceutically acceptable salt thereof, wherein
X² and X⁹, each independently denotes an amino acid residue having a hydrocarbon group optionally having a substituent, N-methyl glycine, 2-azetidine-2-carboxylic acid, proline, thiopurine, 3,4-dehydroproline, pipecolic acid or a derivative thereof.

8. The cyclic peptide according to any one of claims 1 to 7, or a pharmaceutically acceptable salt thereof, wherein X¹⁰ denotes valine, norvaline, leucine, norleucine, isoleucine, methionine, cyclopropyl glycine, cyclobutylglycine, phenylalanine, 4-fluoro-phenylalanine, 2-thienylalanine, 3-thienylalanine, 2-pyridylalanine, 3 2-pyridylalanine, 3-pyridylalanine, 4-pyridylalanine, 2-thiazolylalanine, 4-thiazolylalanine, 2-oxazolylalanine, 4-oxazolylalanine, 1-pyrazolylalanine, 1-imidazolylalanine, 3-(1,2,3-triazol-1-yl)-alanine, 3-(1,2,4-triazol-1-yl)-alanine, 3-(1H-benzoimidazol-1-yl)-alanine, 3-(1,3-benzothiazol-2-yl)-alanine, 3-(1,3-benzoxazol-2-yl)-alanine, 2-quinolylalanine, 8-quinolylalanine, tryptophan, 1-naphthylalanine, 2-naphthylalanine, or their N-methylated amino acid residues, or derivatives thereof.

9. A cyclic peptide comprising an amino acid sequence represented by the following formula (13):
c [X¹-Pro-X³-c(Cys-X⁵-Ser-4fF-Asp-Pro-X¹⁰-X¹¹)] (13)
wherein
X¹ denotes a residue of β-alanine or γ-aminobutyric acid,
X³ denotes a residue of leucine, norleucine, cyclohexylglycine, phenylglycine, 2-aminoheptanoic acid, 2-aminooctanoic acid, 2-aminononanonic acid, or 2-aminodecanoic acid,
X⁵ denotes a residue of isoleucine, norleucine, cyclohexylglycine, phenylglycine, 2-aminoheptanoic acid, 2-aminooctanoic acid, 2-aminononanonic acid, or 2-aminodecanoic acid,
X¹⁰ denotes a residue of valine, phenylalanine, tryptophane or an N-methyl derivative thereof,
X¹¹ denotes a residue of D-cysteine or L-cysteine,
X¹ and X¹¹ form an amide bond between an amino group and a carboxy group in their main chains, and X⁴ and X¹¹ form a covalent bond between their side chain sulfhydryl groups via a linker of a methylene group, an ethylene group, a propylene group or a butylene group, thereby having two cyclic structures in the molecule,
or a pharmaceutically acceptable salt thereof.

10. A cyclic peptide comprising an amino acid sequence represented by the following formula (14):
c[β-Ala-Pro-X³-c(Cys-X⁵-Ser-4fF-Asp-Pro-Trp-^{D}Cys)] (14)
wherein
X³ denotes a residue of leucine, norleucine, cyclohexylglycine, phenylglycine, 2-aminoheptanoic acid, 2-aminooctanoic acid, 2-aminononanonic acid, or 2-aminodecanoic acid,
X⁵ denotes a residue of isoleucine, norleucine, cyclohexylglycine, phenylglycine, 2-aminoheptanoic acid, 2-aminooctanoic acid, 2-aminononanonic acid, or 2-aminodecanoic acid,
residues at position 1 and position 11 form an amide bond between an amino group and a carboxy group in their main chains, and two cysteine resides at position 4 and position 11 form a covalent bond between their side chain sulfhydryl groups via a linker of a propylene group, thereby having two cyclic structures in the molecule,
or a pharmaceutically acceptable salt thereof.

11. A cyclic peptide having an inhibitory activity to Ras, which has an amino acid sequence with deletion, addition and/or substitution of one or several amino acids in the amino acid sequence of the peptide according to any one of claims 1 to 10, or whose amino acid sequence has a sequence identity of at least 50% to the amino acid sequence describes above, and/or whose three-dimensional structure has a structural homology of at least 50% to the peptide according to any one of claims 1 to 8,
or a pharmaceutically acceptable salt thereof.

12. A cyclic peptide having an amino acid sequence represented by any one of SEQ ID Nos. 1 to 21, or
a homologous peptide thereto, wherein one or several amino acids in the amino acid sequence represented by any one of SEQ ID Nos. 1 to 21 are deleted, added and/or substituted, having an inhibitory activity to Ras,
or a pharmaceutically acceptable salt thereof.

13. A derivative or a modification of the peptide according to any one of claims 1 to 12.

14. A medicament, a diagnostic agent and/or a research reagent comprising the peptide according to any one of claims 1 to 13.
